(19) Europäisches Patentamt
European Patent Office
Office européen des brevets

(11) Publication number: 0 068 875 B1

# (12) EUROPEAN PATENT SPECIFICATION

(45) Date of publication of patent specification: 23.12.87

(21) Application number: 82303380.8

(22) Date of filing: 28.06.82

(51) Int. Cl.[4]: C 07 C 101/12, C 07 D 311/16, C 07 D 213/79, C 07 C 157/09, C 07 D 295/14, C 07 D 471/04, C 07 C 103/85 // (C07D471/04, 221:00, 221:00)

## (54) Fluorescent chelates and labeled specific binding reagents prepared therefrom.

(30) Priority: 01.07.81 US 279398

(43) Date of publication of application: 05.01.83 Bulletin 83/01

(45) Publication of the grant of the patent: 23.12.87 Bulletin 87/52

(84) Designated Contracting States: DE FR GB

(56) References cited:
EP-A-0 002 963
DE-A-2 628 158
FR-A-1 242 901
GB-A-2 060 623
US-A-3 312 552
US-A-3 398 099
US-A-3 484 380

CHEMICAL ABSTRACTS, vol. 73, no. 20, 16th November 1970, page 460, no. 105107a, Columbus,Ohio (USA); V. Ya. TEMKINA et al.: "Hydroxystilbene-complexon as a luminescent reagent for scandium"

(73) Proprietor: EASTMAN KODAK COMPANY
343 State Street
Rochester New York 14650 (US)

(72) Inventor: Hinshaw, Jerald Clyde
458 West 4125 North
Ogden Utah 84404 (US)
Inventor: Toner, John Luke
1340 State Road
Webster New York 14580 (US)
Inventor: Reynolds, George Arthur
80 Dunrovin Lane
Rochester New York 14618 (US)

(74) Representative: Baron, Paul Alexander Clifford et al
Kodak Limited Patent Department
Headstone Drive
Harrow Middlesex HA1 4TY (GB)

(56) References cited:
Nicholas J. Turro, Modern Molecular Photochemistry, pages 351-353, The Benjamin/Cummings Publishing Co., Inc.(1978)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 068 875 B1

## Description

The present invention relates to fluorescent chelates and, more particularly, to fluorescent chelates useful for the preparation of specific binding reagents including fluorescent labeled physiologically active materials.

In specific binding assays, sensitivity is of prime importance owing to the generally low analyte levels that are measured.

In fluorescence spectroscopy assays, a sample containing a fluorescent species to be analyzed is irradiated with light of known spectral distribution within the excitation spectrum of the target fluorescent species. The intensity of the resulting characteristic emission spectrum of the fluorescent target molecules is determined and is related to the number of target molecules.

The sensitivity of fluorescence assays, although theoretically very high, is limited by the presence of background fluorescence. Background signal levels are picked up from competing fluorescent substances, not only in the sample, but also in materials containing the sample. This is an especially serious problem in quantitative measurements of species associated with samples containing low concentrations of desired target fluorescent molecules, such as are found in biological fluids. In many situations, it is impossible to reduce the background sufficiently (by appropriate filtration and other techniques known in the art) to obtain the desired sensitivity.

Time resolution offers an independent means of isolating the specific fluorescent signal of interest from nonspecific background fluorescence. Time resolution is possible if the label has much longer-lived fluorescence than the background, and if the system is illuminated by an intermittent light source such that the long-lived label is measureable during the dark period subsequent to the decay of the short-lived background. Such techniques are described in greater detail in German Offenlegungsschrift 2,628,158.

The long-lived fluorescence (0.1—5 msec) of the aromatic diketone chelates of certain rare-earth metals, for example, europiumbenzoylacetonate and europiumbenzoyltrifluoracetonate, has been known for some time. The chelating agent absorbs light and transfers it to the metal ion, which fluoresces. German OLS 2,628,158 describes the use of time resolution in fluorometric immunoassays (FIA) through the use of fluorescent labels whose emissions are long-lived as compared with those of species which produce background interferences in such assays. This publication also provides a useful discussion of the techniques of FIA and its advantage over other immunoassay techniques such as radioimmunoassay (RIA).

The fluorescent immunoreagents described in German OLS 2,628,158 comprise at least one member of the immune system, i.e., an antibody or an antigen, "conjugated" with a rare-earth chelate. Such "conjugation" can be achieved in one of two ways:

(1) first, by labeling, i.e., attaching the rare-earth chelate to the antigen as described in *Fluorescent Antibody Techniques and Their Application* by A. Kawamura, Ed., University Park Press, Baltimore, Maryland, 1969, and then adding antibody to the conjugated antigen whereby the antibody and antigen join in the usual fashion, or:

(2) by covalently bonding the antibody to the chelate via a chemical group.

The problem with immunoreagents of the type described in German OLS 2,628,158 is that the fluorescent labeling species, namely, the rare-earth chelates, are quenched, i.e., their fluorescence is extinguished, when contacted with water. This problem, hereinafter referred to as an "aqueous stability" problem, is particularly serious because a principal use for fluorescent labeled immunoreagents is in the assay of aqueous biological liquids, such as blood and serum. If aqueous stability could be conferred on these materials, they would be useful as fluorescent labels for these biological liquids, thus allowing increased fluorescence immunoassay sensitivity by the use of time resolution of signal from background.

In *Chem. Abst.*, Vol. 73, No. 20, page 460, No. 105107a, there is described a complex of scandium and a stilbene compound. Incidentally, there is mentioned the possible presence of cerium in one of the solutions. However, the described stilbene is not a complexing agent within the scope of the present invention nor would a cerium complex of this complexing agent be formed under the described conditions.

GB—A—2,060,623 describes species-linked diamine triacetic acids which are effective chelating ligands capable of forming metal ion complexes with a variety of metals e.g. lanthanide metals. When used in fluoroassay techniques, the complexes are used in combination with a large excess of a flooder in order to produce the desired fluorescence.

In US—A—3,312,552 there is described the use of certain complexing agents, incidentally falling within the scope of the complexing agents useful in the present invention, which are used in photographic silver halide emulsions. The present invention relates to a field of art which is completely unrelated to silver halide photographic systems. No rare-earth chelates with these complexing agents are described in this reference.

Rare-earth chelates previously used for fluorometric measurements have had undesirable properties, such as a low quantum yield for emission, undesirable sensitizer extinction coefficients which result in insufficient fluorescence using small quantities of detectable species, low $\lambda max$ which renders the determination subject to interference from other components in the sample that are usually in the low $\lambda max$ range, poor water solubility (most biological fluids are aqueous), and poor stability of the chelate at low concentrations.

The present invention is directed to fluorescent chelates of a lanthanide metal and a chelating agent,

the chelating agent including a moiety that is a triplet sensitizer having a triplet energy greater than that of the lanthanide metal and at least two heteroatom-containing groups and a third heteroatom-containing group or heteroatom that is in or appended to said moiety, each of said heteroatom-containing groups appended to different carbon atoms of the triplet sensitizer moiety, the heteroatom-containing groups being located in the chelating agent such that they and said third heteroatom or heteroatom-containing group form a chelate structure with the lanthanide metal.

The chelates are water-soluble, stable at low concentrations at a pH in the range of 8 to 10, highly sensitive, and have favorable molar extinction coefficients (10,000—40,000), and favorable λmax e.g., greater than 300 nm and, preferably, greater than 330 nm, which permits use of a white light source. These chelates have binding constants greater than $10^{10}$ $M^{-1}$. Accordingly, the present invention provides a class of highly efficient, aqueous-stabilized fluorescent chelates for labeling physiologically active materials, such as antigens and hormones. The present invention also provides a new class of specific binding reagents, such as antigens, enzymes, and hormones, bearing these highly useful fluorescent labels.

The fluorescent chelates of the present invention exhibit satisfactory aqueous stability, that is, they exhibit a binding constant greater than $10^{10}$ $M^{-1}$ (i.e. the $\log_{10}$ of the binding constant is greater than 10). This binding constant refers to the binding of the chelating agent to the lanthanide metal.

The reagents are formed by adsorbing or covalently binding the fluorescent labeled antigens, haptens, antibodies, plant lectins, carbohydrates, hormones, enzymes and other such species-specific materials.

Generally, any lanthanide metal is useful in the chelates described herein. Examples of lanthanide metals useful herein are europium and terbium and are described by Sinha, S. P., *Complexes of Rare Earths*, Pergamon Press, 1966.

The sensitizer moiety of the chelating agent is any triplet sensitizer having the requisite triplet energy. Examples of triplet sensitizers useful herein include ketones, such as benzophenone, propiophenone, Michler's ketone, acetophenone, 1,3,5-triacetylbenzene, isobutyrophenone, 1,3-diphenyl-2-propanone, triphenylmethyl phenyl ketone, 1,2-dibenzoylbenzene, 4,4'-dichlorobenzophenone, 1,4-diacetylbenzene, 9-benzoylfluorene, *p*-cyanobenzophenone, β-naphthyl phenyl ketone, 2-acetonaphthone, α-naphthyl phenyl ketone and 1-acetonaphthone, including α,β-diketones such as biacetyl, benzil and 2,3-pentanedione; a ketoaromatic compound, such as xanthone, thioxanthone, anthraquinone, α-naphthoflavone, flavone, 5,12-naphthacenequinone and fluorenone; an aldehyde, such as benzaldehyde, phenylglyoxal, ethyl phenylglyoxalate, 2-naphthaldehyde and 1-naphthaldehyde; a linear or fused polycyclic aromatic compound, such as fluorene, triphenylene, phenanthrene, naphthalene and pyrene; heterocyclic and aromatic nitrogen-containing compounds, such as carbazole, terpyridines, phenanthroline, triphenylamine, thiazolines, especially 2-organocarbonylthiazolines, such as 2 - benzoylmethylene - 1 - methylnaphtho[1,2 - d] - thiazoline, 2 - furoylmethylene - 1 - methylnaphtho[1,2 - d] - thiazoline, 2 - (difuroylmethylene) - 1 - methylnaphtho[1,2 - d] - thiazaline, 1 - methyl - 2 - thenoylmethylenenaphtho[1,2 - d] - thiazoline and 2 - (dithenoylmethylene) - 1 - methylnaphtho[1,2 - d] - thiazoline; thiazoline compounds, as described in U.S. Patents 2,732,301 and 4,119,466; and ketocoumarins, such as described in U.S. Patent 4,147,552.

When the lanthanide metal is europium, the triplet energy must be at least 47 Kcal, and if the lanthanide metal is terbium, the triplet energy of the nucleus must be at least 53 Kcal.

The chelating agent also has at least two heteroatom-containing groups that are located on the chelating agent such that they are capable of forming coordinate bonds with lanthanide metals. Groups capable of forming coordinate bonds with lanthanide metals include nitrilodiacetate, carboxy, hydroxy, alkoxy, amino, amido, carbonyl, and mercapto groups. These groups are appended to the nucleus so as to allow chelation of the groups with the lanthanide metal.

Preferred chelating agents have the structure:

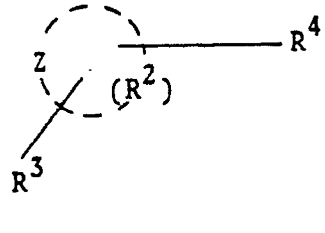

wherein:

Z together with $R^2$ represents the atoms necessary to complete a substituted (such as with a group used to link up the immunoreagent such as ureylene, thioureylene, carbonylimino or imino linked to an immunoreagent, or a coumarin group) or unsubstituted moiety that is a triplet sensitizer having a triplet energy greater than that of the lanthanide metal;

$R^2$ is (1) a heteroatom or (2) an alkylene (including alkenylene) group having at least one heteroatom therein or a heteroatom or heteroatom-containing group appended thereto; and

$R^3$ and $R^4$ are heteroatom-containing groups that are the same or different; $R^3$ and $R^4$ being in sufficient proximity to $R^2$ so that the lanthanide metal is chelatable to $R^2$, $R^3$ and $R^4$; wherein the number of carbon and heteroatoms represented by $R^2$ is equal to or less than 20.

$R^2$ is a heteroatom, such as nitrogen, oxygen, sulfur, or selenium, or an alkylene (including alkenylene) group having therein at least one heteroatom or a heteroatom or heteroatom-containing group appended thereto. Thus, $R^2$ can comprise one or more heteroatoms. Examples of $R^2$ are

-NH-, O, S, Se, -N=,

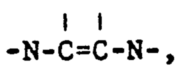
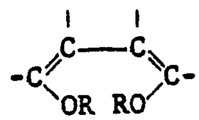
(R = H, aryl or alkyl),
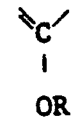

and a heteroatom or a heteroatom-containing alkylene group of up to 10 carbon atoms. Further examples are carbonyl, dicarbonyl, thiocarbonyl, hydroxymethylene, 1,2-dihydroxyethylene and 1,2-dihydroxyvinylene.

It is preferred that $R^3$ and $R^4$ be either individually adjacent to $R^2$, or three or less atoms removed from $R^2$.

In one preferred embodiment, the lanthanide metal is chelated with a phenol having iminodiacetate groups substituted in each position ortho to the phenolic hydroxy group. The phenol is unsubstituted or substituted with a variety of groups, such as alkoxy, alkyl, halogen, or carbonyl, and is optionally fused to another aromatic group or to an alicyclic or heterocyclic group. Especially preferred are compounds having the structure:

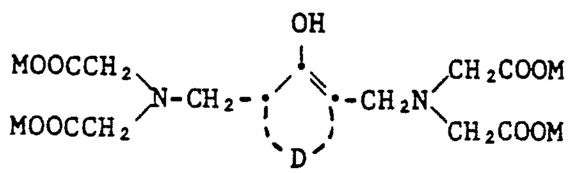

wherein:

M is hydrogen or a cation, such as ammonium or its derivatives, such as tetramethylammonium, tetraethylammonium, or benzyltrimethylammonium, or an alkali metal, such as sodium, lithium, potassium, rubidium, or cesium; and

D represents the atoms necessary to complete a substituted or unsubstituted aromatic ring. This aromatic ring must bear a hydroxyl group as shown above. In addition, it must bear a carbonyl group such as

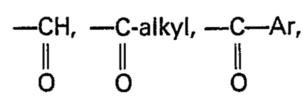

where alkyl generally contains up to 10 carbon atoms, such as methyl, ethyl, propyl, butyl, pentyl, hexyl, heptyl, octyl, nonyl, decyl, or isomers thereof and Ar is aryl, such as phenyl; or it must be fused at two of its available positions to another aromatic, alicyclic, or heterocyclic ring preferably containing from 4 to 7 carbon atoms, such as benzene (substituted or unsubstituted), benzophenone, or pyran which bears a carbonyl group to form a coumarin nucleus. Examples of the aromatic ring are phenyl, naphthyl, and the like, optionally substituted in any of the available positions with alkyl, preferably containing from 1 to 4 carbon atoms, such as methyl, ethyl, propyl, or butyl or isomers thereof; hydroxy; aryl, such as phenyl; aldehyde groups such as CHO; or benzoyl groups, such as:

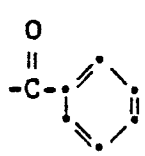

In especially preferred embodiments, D completes a phenyl ring with a carbonyl substituent, such as a benzoyl substituent or it completes a coumarin group. Throughout the specification the terms "alkyl" and "aryl" include substituted alkyl and aryl. Particularly useful substituent groups include methyl, ethyl, and propyl.

A preferred embodiment of the invention involves the formation of a chelate of a lanthanide metal with a salt or acid having the following structure:

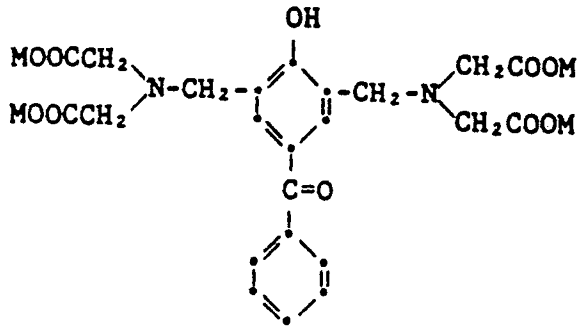

wherein:

M is hydrogen, ammonium, or an alkali metal ion, or any other suitable cation that renders the salt water-soluble.

In another preferred embodiment, the organic compound that complexes with the lanthanide metal has the following structure:

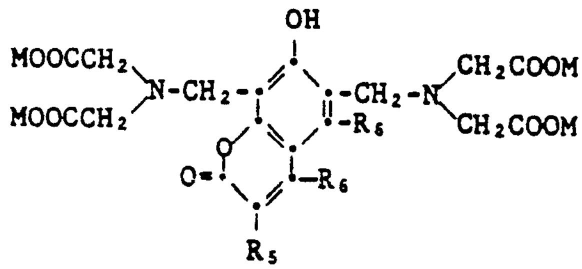

wherein:

$R_5$ is preferably aroyl such as:

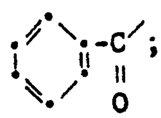

and

each $R_6$ is independently selected from hydrogen; alkyl having from 1 to 4 carbon atoms, such as methyl, ethyl, propyl, or butyl; aryl or aroyl with or without further substitution; alkoxy, such as methoxy and propoxy; or halogen, such as bromine or chlorine.

The preferred aroyl $R_5$ substituent is optionally further substituted with aryl or alkyl groups, or with ester, amide, carbamide, thiocarbamide, isocyanate, thiocyanate, halogen, or nitrile groups.

Certain other coumarin compounds in which $R_5$ is appended to the coumarin ring by other than an electronegative (i.e., an electron-withdrawing) group are known to fluoresce intensely but are not as useful in the practice of this invention, as this fluorescence prevents the transfer of energy of excitation to the europium or terbium complex with subsequent fluorescence in the visible portion of the spectrum. The organic salt or acid used to form the rare-earth chelate must absorb in the region of 300—500 nm and must then transfer its excitation energy to the lanthanide metal, which then fluoresces in the visible portion of the spectrum. Other examples of useful complexing compounds include:

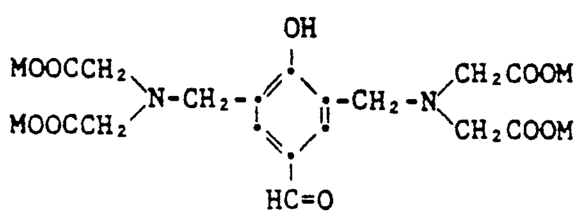

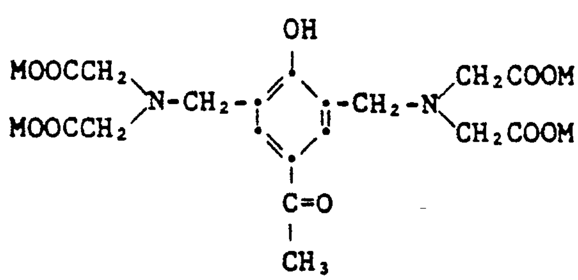

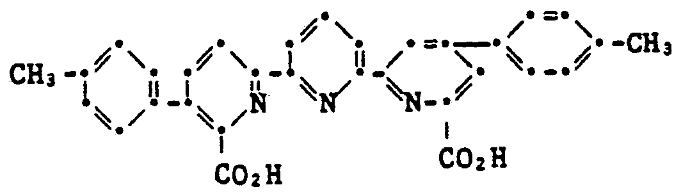

and

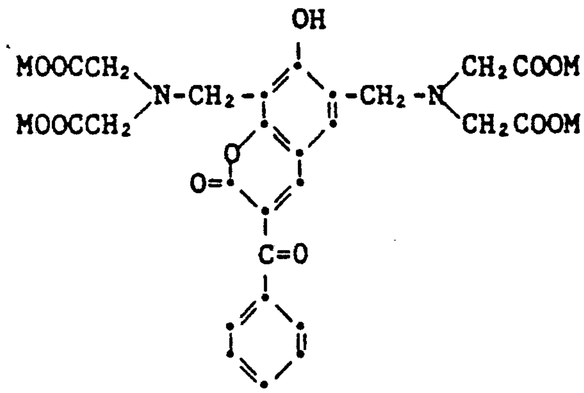

The complex contains any ratio of lanthanide metal to chelating agent. In preferred embodiments, the mole ratio of lanthanide metal to chelating agent is from 1:1 to 2:1, most preferably, 1:1.

The chelating agents are prepared by performing a Mannich reaction between known compounds of the structure

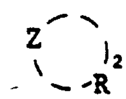

and iminodiacetic acid or esters thereof, and formaldehyde; or by a nucleophilic displacement reaction between compounds of the structure

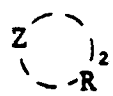

that have active methylene groups, such as bromomethyl or methylenetosylate groups, and iminodiacetic esters, and in a subsequent step, hydrolyzing the esters.

Useful complexes include

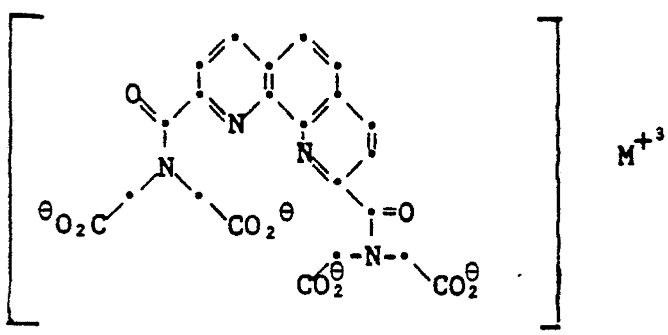

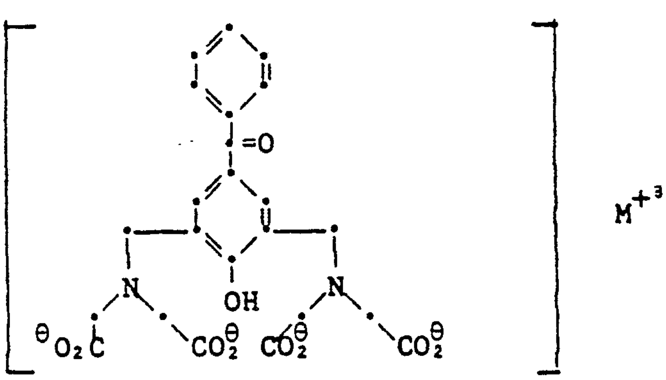

and

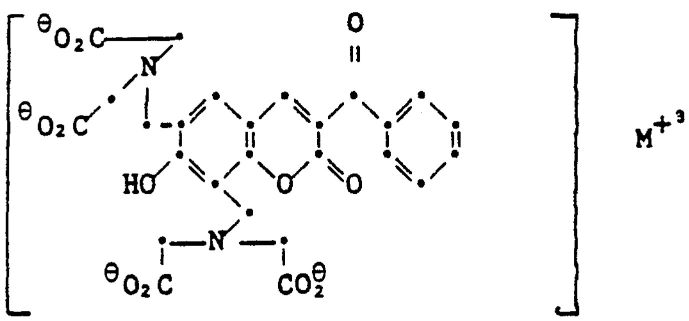

The lanthanide metal and the chelating agent are easily complexed by merely mixing an aqueous solution of the chelating agent with a lanthanide metal salt in an aqueous solution of pH 7.5—10. The lanthanide metal salt is any water-soluble salt of the metal, such as chloride salts, such as $TbCl_3 \cdot 6H_2O$ or $EuCl_3 \cdot 6H_2O$. The present complexes maintain electrical neutrality by virtue of the presence of another cation in the buffer solution.

The chelate is generally prepared in aqueous solution at a pH of from 8 to 11, preferably, 8 to 9.

The chelate optionally is mixed with buffers such as phosphate and borate, to produce the optimum pH.

The chelate is useful to label a variety of physiologically active materials by binding them to the complex by adsorption or by covalent bonding. Among the physiologically active materials that are labeled in this fashion are enzymes and their substrates, antigens, i.e., any substance that is capable, under appropriate conditions, of reacting specifically in some detectable manner with an antibody, carbohydrate, metabolites, drugs, other pharmacological agents and their receptors and other binding substances. Specific binding assay reagents are described in U.S. Patents 3,557,555, 3,853,987, 4,108,972 and 4,205,058.

Techniques for performing such binding of physiologically active materials to the complexes are well-known in the art and include simply mixing the materials together.

In specific binding assay methods, a compound having structural similarity to the analyte being determined is bonded to a detectable label. The analyte being determined is herein referred to as the ligand and the labeled compound as the ligand analog. Compounds that specifically recognize the ligands and ligand analogs and bind to them are referred to as receptors.

In performing one such type of assay, the ligand is placed in competition with the ligand analog for binding to the receptor. Unknown concentrations of the ligand are inferred from the measured signal of the labeled, ligand analog. The reaction proceeds as follows:

$$\text{ligand} + \text{(labeled) ligand analog} + \text{receptor} \rightleftharpoons \text{ligand/receptor} + \text{ligand analog/receptor}$$

For illustrative purposes, the discussion that follows describes one particular type of specific binding assay technique, a competitive binding fluorescence immunoassay technique.

This system consists of antigen labeled with a fluorescent label, unlabeled native antigen (in test sample) and specific antibody whereby there is competition between the unlabeled antigen and the labeled antigen for binding to the antibody.

The greater the concentration of unlabeled antigen from the test sample in the system, the less the labeled antigen will be bound by the antibody. If the concentration of labeled antigen and antibody is fixed and the only variable is the level of unlabeled antigen, it is possible to determine the unknown level of unlabeled antigen by physically separating the antigen-antibody complex from the remaining free antigen (both labeled and unlabeled) and comparing the fluorescence of the labeled antigen, either free *or* bound, with a standard curve plotting of the values given by a range of known amounts of the antigen treated in the same manner.

A preferred fluorescently labeled specific binding reagent comprises a complex of a lanthanide metal and a chelating agent having the structure:

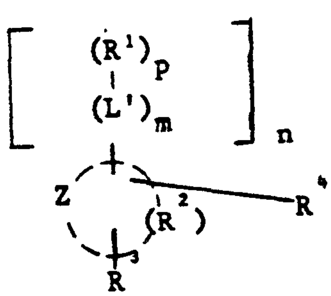

wherein:

Z, $R^2$, $R^3$, and $R^4$ are as described above and L′ is a linking group, such as an ester, such as

$$-\overset{O}{\overset{\|}{C}}O-,\ -\overset{S}{\overset{\|}{C}}O-,\ -\overset{S}{\overset{\|}{C}}-S-;$$

amide, such as

$$-\overset{O}{\overset{\|}{C}}NH-,\quad -\overset{O}{\overset{\|}{C}}-\overset{C_2H_5}{\overset{|}{N}}-;$$

sulfonamide, such as

$$SO_2NH-,\ SO_2\overset{C_2H_5}{\overset{|}{N}}-;$$

ether, such as —O—, —S—; carbonyl, such as

$$-\overset{O}{\overset{\|}{C}}-,\ -\overset{S}{\overset{\|}{C}}-;$$

nitrilo, such as =N—; and imino, such as —NH—; including those groups comprising additional organic linking atoms, such as arylene and thioarylene;

p is 1, m is 0 or 1, n is 1 to 3, and $R^1$ is a physiologically active material, such as an antigen or hormone.

Once prepared as described hereinabove, the fluorescent-labeled, physiologically active species is useful in fluorescent specific binding assays, particularly those that utilize temporal resolution of the specific detecting signal to distinguish from background as described in German OLS 2,628,158. In this time-resolved mode (i.e., temporal resolution), the sample is excited in an intermittent fashion and information is accepted only during the dark cycle when the long-lived fluorescent label is still emitting strongly, but when other sources of fluorescence have decayed. Discontinuous excitation is achieved in a variety of ways, including pulsed laser, mechanical chopping of a continuous excitation beam, and moving the sample in and out of the excitation beam. Moreover, discontinuous excitation has the advantage of allowing the use of high radiant power without the absorption of a large amount of energy by the sample, thus diminishing the probability of sample photodegradation.

Examples of such fluorescent specific binding assay techniques wherein the specific binding reagents described herein find utility are described in U.S. Patents 3,998,943, 4,020,151, 3,939,350, 4,220,450 and 3,901,654.

Especially preferred fluorescently labeled specific binding reagents include those of the following structures:

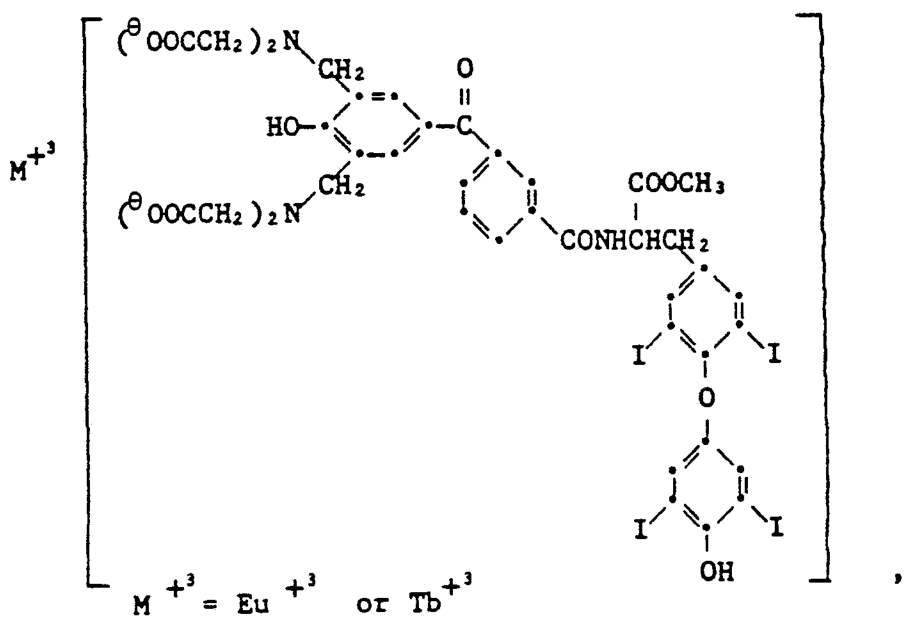

$M^{+3} = Eu^{+3}$ or $Tb^{+3}$

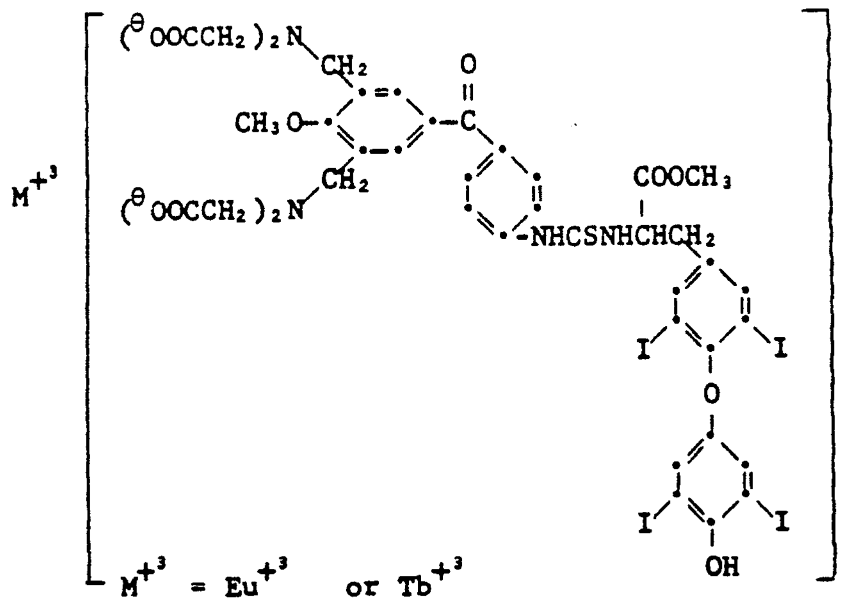

$M^{+3} = Eu^{+3}$ or $Tb^{+3}$ ,

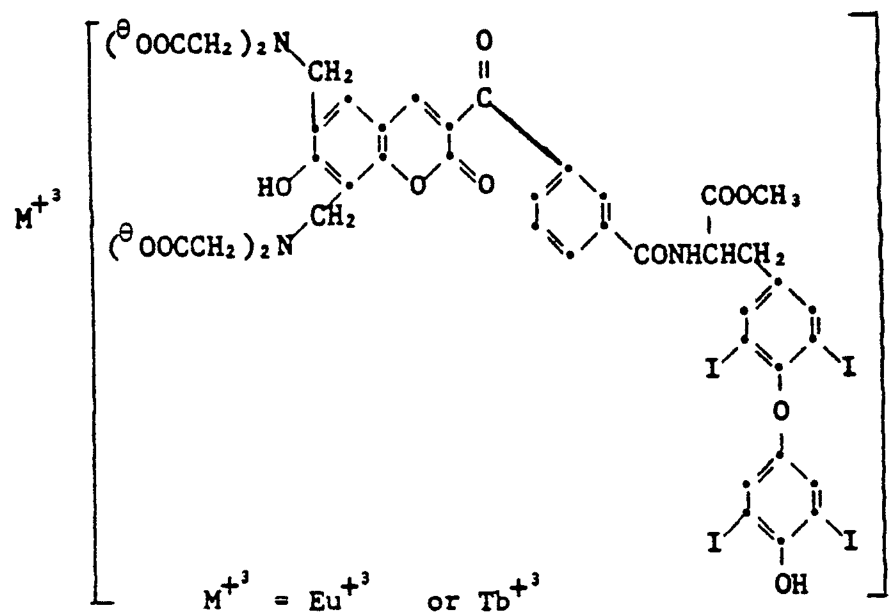

$M^{+3} = Eu^{+3}$ or $Tb^{+3}$ ,

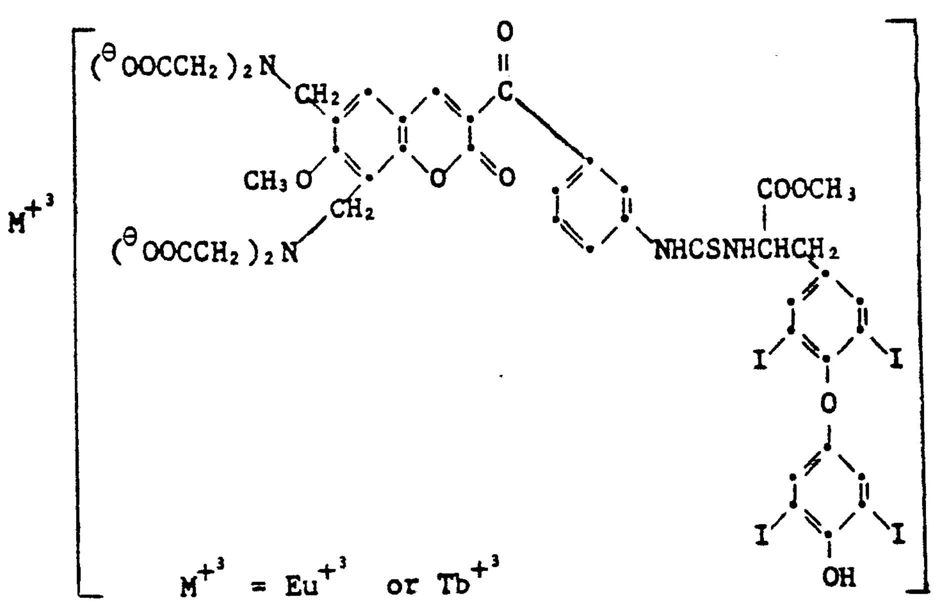

$M^{+3} = Eu^{+3}$ or $Tb^{+3}$ and

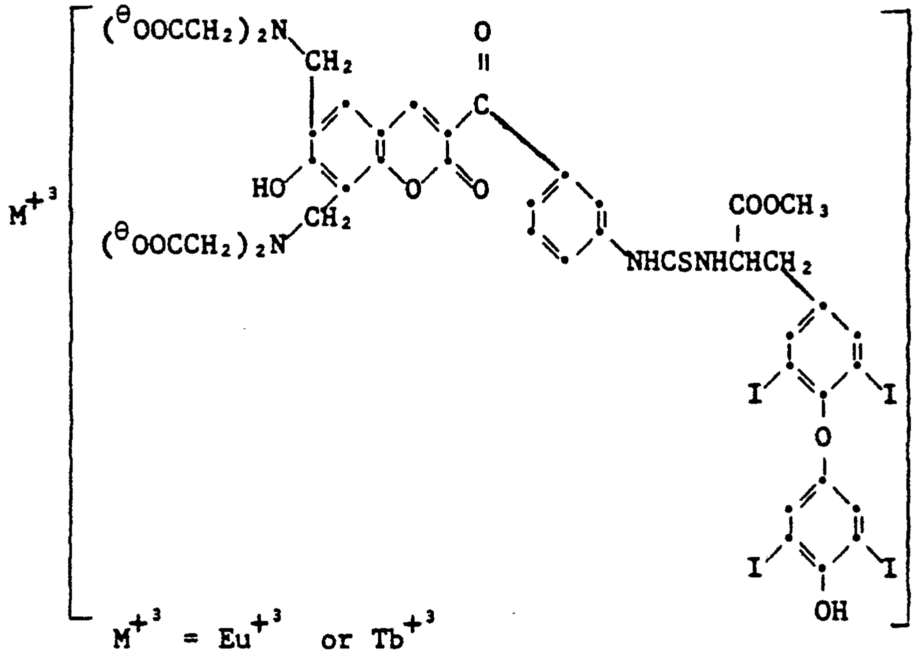

$M^{+3} = Eu^{+3}$ or $Tb^{+3}$

In a preferred embodiment, the specific binding assay is carried out in a dry analytical element, such as is described in U.S. Patent 4,258,001. In this embodiment, the element contains a support and a spreading/reagent layer comprised of polymeric beads, and, optionally, a registration layer. In some cases, the spreading layer is separate from the reagent layer. The spreading, reagent, and registration layers optionally comprise the polymeric bead structure. The polymeric beads of the reagent layer have receptors such as antibodies adsorbed to their surfaces.

The chelate label is placed above, below, or in the reagent layer in a manner that prevents the specific reaction from occurring prior to sample wetting, or it is spotted onto the element concurrently with or subsequent to the sample. It is only necessary that the labeled ligand analog permeate the element upon wetting subsequently to compete with the unknown amount of ligand in the sample in the formation of the ligand-receptor complex. The assay is performed by fluorimetrically determining the amount of free labeled ligand analog present or the amount of bound labeled ligand analog-receptor complex.

The following nonlimiting examples will serve better to illustrate the successful practice of the instant invention.

Example 1

A complex was formed by mixing equimolar amounts of $TbCl_3 \cdot 6H_2O$ in an aqueous solution containing a borate buffer, which results in a pH of 9, with a compound having the structure:

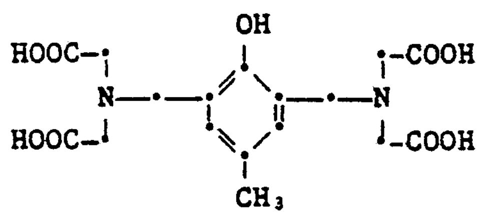

which was prepared by the method described by G. Schwarzenbach et al, *Helv. Chim. Acta, 35,* 1785 (1952). A 37% aqueous formaldehyde solution (35.6 g, 0.44 mol) was added dropwise to a solution of p-cresol (21.6 g, 0.20 mol) and imino-diacetic acid (56.0 g, 0.42 mol) in 90 ml of $H_2O$ and 88 ml of concentrated aqueous NaOH at 10—20°C. The resulting mixture was heated at 60—70°C. for 2 hours, then neutralized with 86 ml of concentrated HCl. The solvent was removed *in vacuo* and the residue was heated in 1200 ml of ethanol. The insoluble material was removed by filtration and the solvent volume was reduced to 600 ml and cooled. The precipitated salts were removed by filtration and the filtrate was evaporated to dryness to give 79 g of 2,6-bis[N,N-bis(carboxymethyl)aminomethyl]-4-methylphenol as a white powder (99%). The product was recrystallized from ethanol. A complex was formed by mixing equimolar amounts of the product and $TbCl_3$—$6H_2O$ in aqueous borate buffer at pH 9. A bright green emission was shown when the solution was excited with a long-wavelength UV lamp (Model UVL—21 Blak Ray (trade mark) lamp having a λmax at 366 nm).

Example 2

To a stirred solution of 9.9 g (.05 mole) of *p*-hydroxybenzophenone and 13.7 g (0.1 mole) of iminodiacetic acid in 60 ml of water containing 9 g of sodium hydroxide was added slowly 8.9 g of 37% aqueous formaldehyde solution. The mixture was stirred and refluxed for 5 hours, then cooled to room temperature, and brought to pH 2 with 2N hydrochloric acid. The solid that formed was collected, washed with water, and air-dried. The product was recrystallized from 500 ml of 90 percent ethyl alcohol to give 5.9 g of white-to-pinkish solid in two crops.

Anal. calcd. for
$C_{23}H_{24}N_2O_{10}\cdot H_2O$: C, 54.5; H, 5.2; N, 5.5.
Found: C, 54.5; H, 4.8; N, 6.1.

UV spectrum (pH 9 borate buffer) λmax 320 nm, ε $1.6 \times 10^4$.

An exactly equimolar mixture of the above compound and $EuCl_3\cdot 6H_2O$ or $TbCl_3\cdot 6H_2O$ in pH 9 borate buffer showed a bright red ($Eu^{+3}$) or green ($Tb^{+3}$) fluorescence when excited with a long-wavelength ultraviolet lamp. The emission intensity from the above europium chelate solution was examined as a function of concentration on a Farrand (trade mark) Spectrofluorimeter sold by Farrand Instrument Company. The data displayed a linear decrease in the logarithm of the emission as a function of the logarithm of the concentration from $10^{-5}$ to $10^{-9}$ M in europium chelate. The fluorescence quantum yield (ϕ) for a $10^{-6}$ M solution of the $Eu^{+3}$ complex in pH 8.5 borate buffer excited at λ = 320m was 0.003. A similar solution of the $Tb^{+3}$ complex had ϕ = 0.10. The $\log_{10}$ values of the binding constants of this ligand toward $Eu^{+3}$ and $Tb^{+3}$ in water were 16.70 ± 0.12 $M^{-1}$ and 16.78 ± .11 $M^{-1}$, respectively.

Example 3

To a solution of 1.64 g (0.02 mole) of 37 percent aqueous formaldehyde in 25 ml of methanol was added 3.22 g (0.02 mole) of dimethyl iminodiacetate. The solution was concentrated under reduced pressure on a rotary evaporator. Methanol (25 ml) was added to the residue and the solution was again concentrated. To the remaining oil was added 2.66 g (0.01 mole) of 3-benzoyl-7-hydroxycoumarin, followed by 4 ml of N-methylmorpholine. The mixture was heated with stirring at 115°C for 3 hrs. and then concentrated under reduced pressure on a rotary evaporator. The resulting thick oil was dissolved in a minimum of $CH_2Cl_2$ and applied to a dry column of silica gel. The column was eluted with 1:4 ethyl acetate:$CH_2Cl_2$. The first yellow band of monoadduct was discarded. The second yellow band was collected. Removal of the solvent under reduced pressure gave 1.6 g of the desired product as a yellow oil that could not be induced to crystallize.

To a solution of 1.5 g (.0027 mole) of the above tetraester in 20 ml of acetic acid was added 0.6 g (.003 mole) of cupric acetate monohydrate, followed by 10 ml of water. The mixture was stirred and refluxed under nitrogen for 2 hours. The reaction was cooled to room temperature and brought to about pH 2 with hydrochloric acid. With stirring, the mixture was saturated with hydrogen sulfide gas and allowed to stand 15 minutes. The precipitated cupric sulfide was removed by suction filtration through a diatomaceous-earth pad. The clear orange-brown filtrate was concentrated under reduced pressure on a rotary evaporator. The residue was dissolved in hot ethanol containing 25% water, then allowed to cool finally at 5°C. for several hours. The solid was collected, washed with water and dried *in vacuo* to give 0.3 g of product. UV spectrum (pH 9 borate buffer) λmax 396 nm, ε 27,000.

Analysis calculated for
$C_{26}H_{24}N_2O_{12}$: C, 56.1; H, 4.3; N, 5.0.
Found: C, 55.6; H, 4.2; N, 4.6.

An exactly equimolar amount of the above compound and $EuCl_3\cdot 6H_2O$ in pH 9 borate buffer showed a bright red emission when excited with a long-wavelength ultraviolet lamp.

The emission intensity from the above europium chelate solution was examined as a function of concentration on a Farrand Spectrofluorimeter. The data displayed a decrease in emission as a function of concentration from $10^{-5}$ to $10^{-10}$ M in europium chelate.

λmax emission 593 nm, 614 nm, 652 nm, 701 nm
Emission quantum yield over 560 to 800 nm = 4.5%
Emission quantum yield at 614 nm = 3.7%

The $\log_{10}$ values for the binding constants of this ligand with $Eu^{+3}$ and $Tb^{+3}$ in water were 16.26 ± 0.23 $M^{-1}$ and 16.35 ± 0.21 $M^{-1}$ respectively.

Example 4

Preparation of 2,4-dihydroxy-3,5-bis[N,N-di(ethoxycarbonylmethyl)-aminomethyl]benzaldehyde

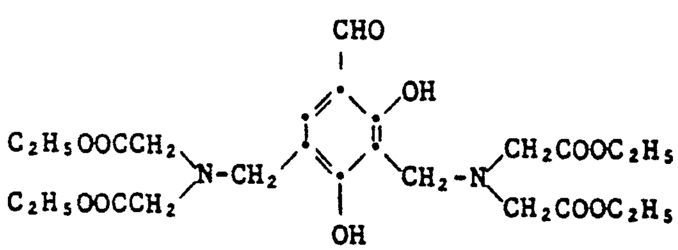

To 8.2 g (0.1 mole) of 37% aqueous formaldehyde in 50 ml of ethanol was added 18.9 g (0.1 mole) of diethyl iminodiacetate. The mixture was concentrated under reduced pressure on a rotary evaporator. An additional 50 ml of ethanol was added and the mixture again concentrated to dryness. To the resulting oil was added 6.9 g (0.05 mole) of solid 2,4-dihydroxybenzaldehyde. The mixture was stirred and heated at 120°C. for 3 hours, then used without purification.

The above was repeated using dimethyl iminodiacetate with similar results.

Example 5

Preparation of 3-(4-nitrobenzoyl)-7-hydroxy-6,8-bis[N,N-di(carboxymethyl)aminomethyl]coumarin

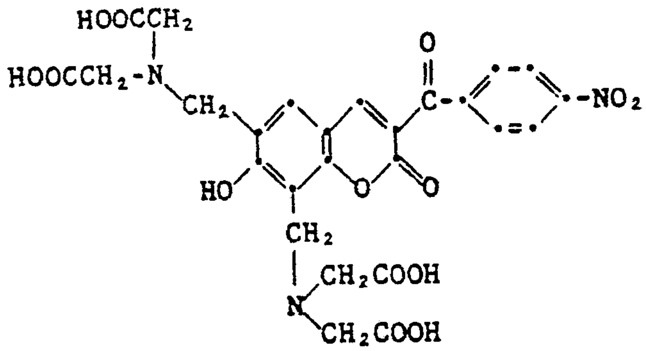

To the above Example 4 crude aldehyde (.05 mole) was added 11.85 g (.05 mole) of ethyl 4-nitrobenzoylacetate, followed by 100 ml of ethanol. A solution of 30 mg of acetic acid and 42 mg of piperidine in 1 ml of ethanol was added, and the mixture was stirred and refluxed for 16 hours. After this time, the reaction product was concentrated on a rotary evaporator. The resulting oil was dissolved in a minimum of $CH_2Cl_2$ and applied to a silica gel dry column. The column was eluted with 150:850 ethyl acetate:$CH_2Cl_2$. The first running colorless impurity and a second running dark-yellow impurity were discarded. The slower-moving light-yellow product band was collected and the solvent was removed under reduced pressure. The resulting oil was analyzed by NMR and mass spectroscopy and used directly. Yield, 14.3 g.

To 2.67 g (.00375 mole) of the tetraester formed in the preceding paragraph in 75 ml of acetic acid was added 1.0 g (.005 mole) of cupric acetate monohydrate, followed by 25 ml of water. The mixture was stirred and refluxed under nitrogen for 2 hours. The reaction was cooled to room temperature and brought to about pH 2 with hydrochloric acid. An excess of hydrogen sulfide gas was then passed into the stirred solution, and the mixture was allowed to stand 30 minutes. The precipitated cupric sulfide was removed by suction filtration through a diatomaceous-earth pad. The filtrate was concentrated to dryness under vacuum on a rotary evaporator. The residue was triturated with 50 ml of water. The solid was collected, washed well with water and dried. Yield, 1.2 g. A sample was dissolved in hot 50% aqueous ethanol. The mixture was concentrated under reduced pressure until a solid formed. The solid was collected, washed with cold water, and dried.

| | | | |
|---|---|---|---|
| Anal. calcd. for $C_{26}H_{23}N_3O_{14}\cdot H_2O$: | C, 50.4; | H, 4.1; | N, 6.8. |
| Found: | C, 50.8; | H, 4.1; | N, 6.4. |

Catalytic reduction of the above chelating agent in aqueous sodium bicarbonate solution gave the corresponding amino compound.

Example 6

A stock solution containing a $10^{-4}$ M concentration of a europium chelate described in Example 3 above was diluted with borate buffer (pH 8.5) to concentrations shown in Table I. Ten-microliter aliquots of each concentration were spotted onto analytical elements prepared in the following manner:

A polycarbonate film support was coated with a microbead layer comprised of poly(styrene-co-methacrylic acid) (weight ratio 98:2) (75.0 g/m$^2$), which had been adsorbed with ovalbumin, carboxymethyl cellulose (0.19 g/m$^2$), Zonyl FSN (trade mark) (a nonionic fluorosurfactant obtained from duPont), 0.05% based on total melt weight, normal rabbit serum (0.83 g/m$^2$), poly(*n*-butyl acrylate-co-styrene-co-2-acrylamido-2-methyl propane sulfonic acid) (weight ratio 70:20:10) (2.25 g/m$^2$) and $H_3BO_3\cdot KCl$ buffer at pH 8.5.

The elements were then evaluated, using a fluorimeter having a Wrattan 18A filter, at Excitation$_{300-400}$ nm and Emmission$_{620}$ nm, at a pH of 8 and a pH of 9.18.

The results shown in the table below illustrate that the fluorescence signal obtained is a function of the concentration of the europium chelate in the sample. The background fluorescence was 50 mV.

TABLE I

| Concentration of Eu Chelate | pH | Fluorescence at $Em_{620}$ nm |
|---|---|---|
| $10^{-8}$ | 8 | 80—100 mV |
| $10^{-7}$ | 8 | 400—450 mV |
| $10^{-6}$ | 8 | 4500 mV |
| $10^{-5}$ | 8 | 40 V |
| $10^{-8}$ | 9.18 | 60 mV |
| $10^{-7}$ | 9.18 | 250 mV |
| $10^{-6}$ | 9.18 | 2.1 V |
| $10^{-5}$ | 9.18 | 20 V |

Example 7

A terbium compound prepared as described in Example 3 was tested in the manner of Example 6. The results are shown in Table II with the fluorescence measured at 550 nm and given in microamperes (μA).

TABLE II

| Concentration of Tb Chelate (M) | pH | Fluorescence at $Em_{550}$ nm (μA) |
|---|---|---|
| $10^{-5}$ | 8.0 | 11.1 |
| $10^{-6}$ | 8.0 | 1.41 |
| $10^{-7}$ | 8.0 | 0.27 |
| $10^{-8}$ | 8.0 | 0.04 |
| $10^{-9}$ | 8.0 | 0.05 |

Example 8

Complex of europium and 3,5-bis[N,N-bis(carboxy-methyl)aminomethyl]-4'-{N'-[4-(4-hydroxy-3,5-diiodo-phenoxy)-3,5-diiodo-β-methoxycarbonyl-phenethyl]thioureido}-4-methoxybenzophenone

The synthetic scheme for the preparation of the chelating agent is as follows:

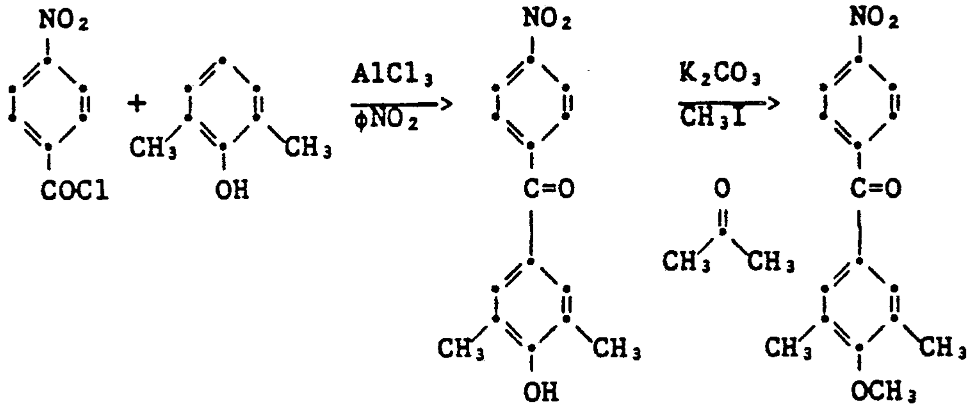


1 2

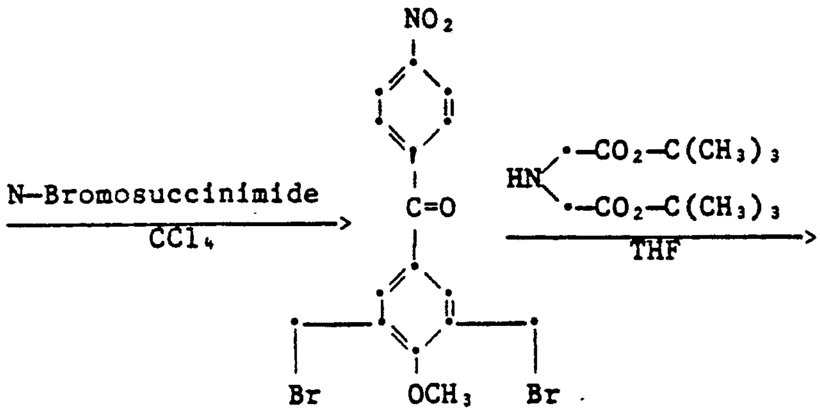


3

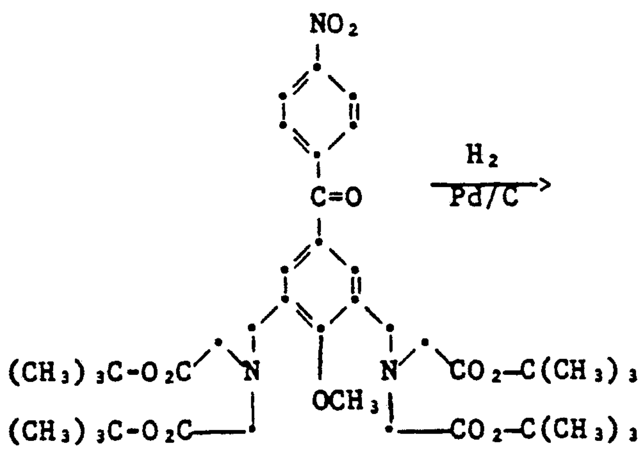


4

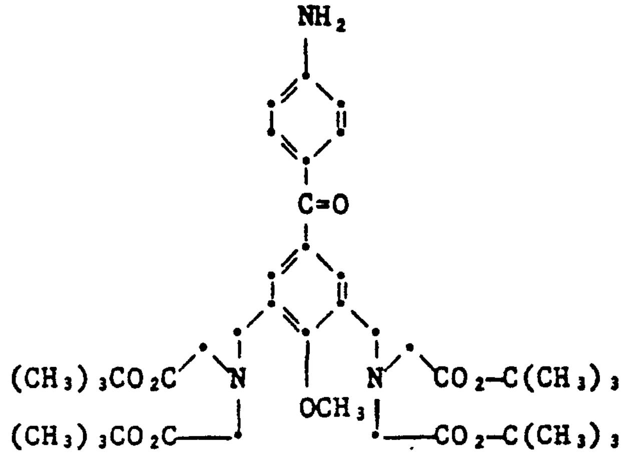

5

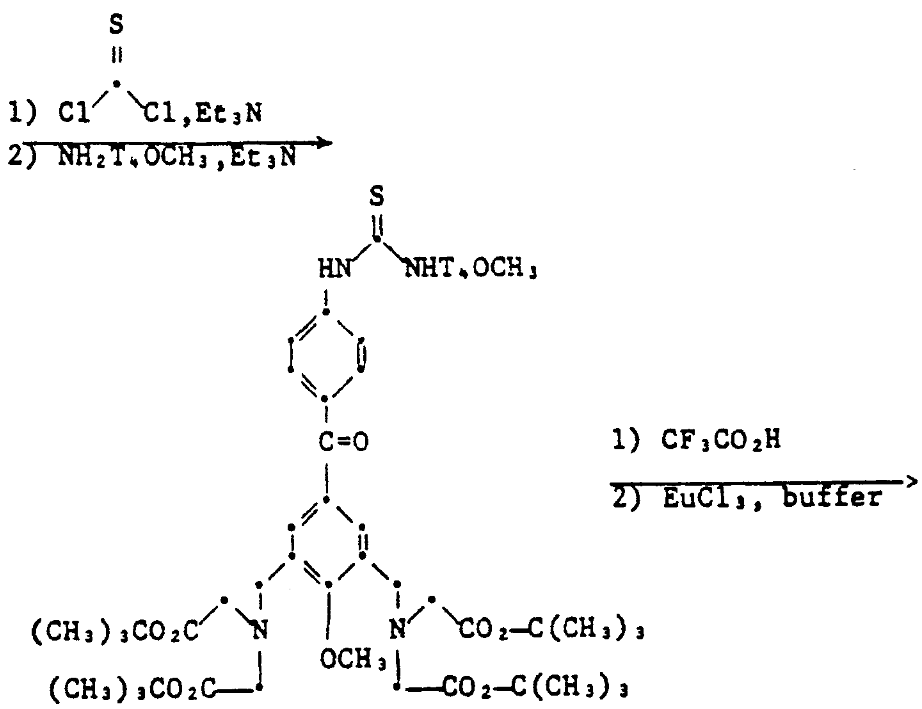


6

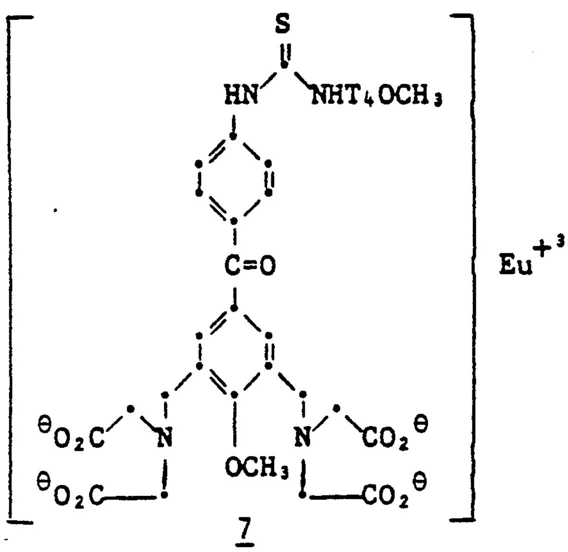

7

where $NH_2T_4OCH_3$ is

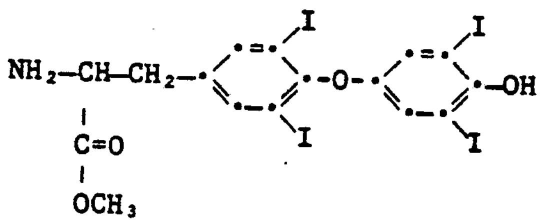

Preparation of 3,5-dimethyl-4-hydroxy-4'-nitrobenzophenone (1)

To a stirred solution of *p*-nitrobenzoyl chloride (76.1 g, 0.410 mole) in 250 mL nitrobenzene was added 82 g of $AlCl_3$ (0.62 mole). To this mixture, a solution of 50 grams of 2,6-dimethylphenol (0.41 mole) in 250 ml nitrobenzene was added dropwise over a period of 45 minutes, and the resulting mixture was stirred for 16 hours at room temperature. The reaction was poured into 2 litres of 3% HCl and ice and extracted 3 × 1 litre with $(CH_3CH_2)_2O$ (i.e., three times with 1 litre each time of diethyl ether). The combined ethereal layers were washed with 1 litre of saturated $NaHCO_3$, then extracted 2 × 1 litre with 10% NaOH. The combined basic extracts were acidified with concentrated HCl to give a white precipitate; filtration followed by recrystallization from $CH_3OH/H_2O$ gave 37 g (33%) of white powder consistent with the desired product by thin layer chromatography (TLC), IR, mass spectroscopy, NMR, and elemental analysis, m.p. 182.5—184.5.

Anal. Calcd. for

| | | | |
|---|---|---|---|
| $C_{15}H_{13}NO_4 \cdot H_2O$: | C, 62.27; | H, 5.24; | N, 4.84. |
| Found: | C, 62.34; | H, 5.31; | N, 4.76. |

Preparation of 3,5-dimethyl-4-methoxy-4'-nitrobenzophenone (2)

A solution containing compound *1* (15.0 g, 55.4 mmol), $K_2CO_3$ (20.0 g, 0.145 mole) and $CH_3I$ (30 ml, 0.48 mole) in 250 ml acetone was refluxed for 6 hours. The solvent was evaporated and the residue partitioned between $CH_2Cl_2$ and $H_2O$. The organic layer was dried over $Na_2SO_4$, filtered, and evaporated to leave a yellow-white powder. Recrystallization from $CH_3OH$/petroleum ether at −20°C gave 14.9 g of white crystals (94%). The material was characterized by NMR, TLC, mass spectroscopy, IR, and elemental analysis, m.p. 131—132.5°C.

Anal. Calcd. for

| | | | |
|---|---|---|---|
| $C_{16}H_{15}NO_4$: | C, 67.35; | H, 5.31; | N, 4.91. |
| Found: | C, 67.54; | H, 5.38; | N, 4.87. |

Preparation of 3,5-bisbromomethyl-4-methoxy-4'-nitrobenzophenone (3)

A mixture of compound *2* (4.0 g, 14 mmol) and *n*-bromosuccinimide (5.0 g, 28 mmol) was refluxed in 250 ml $CCl_4$ with *ca.* 50 mg dibenzoyl peroxide as a radical initiator for 1 hr. under $N_2$. The reaction was cooled to room temperature and 100 ml $CH_2Cl_2$ was added. Filtration followed by extraction of the filtrate with aqueous sodium thiosulfate, drying the organic layer over $Na_2SO_4$, filtration, and solvent removal left a white powder. This powder was triturated 3 × 50 ml with $(CH_3CH_2)_2O$ to leave 5.7 g of white powder that TLC showed contained 3 components. NMR and mass spectroscopy confirmed the presence of impurities, but the majority of the material was the desired product.

Preparation of 3,5-bis[N,N-bis(t-butoxycarbonylmethyl)-aminomethyl]-4-methoxy-4'-nitrobenzophenone (4)

A solution of compound *3* (2.0 g, 4.5 mmol) and di-*tert*-butyl iminodiacetate (2.2 g, 9.0 mmol) was stirred in 200 ml of tetrahydrofuran for 60 hrs. at room temperature. The solvent was removed and the remaining yellow oil was partitioned between $CH_2Cl_2$ and cold aqueous $K_2CO_3$ made from highly purified water. The organic layer was dried over $Na_2SO_4$, filtered, and evaporated to leave 4.5 g of yellow oil. A preparative gel permeation column with $CH_2Cl_2$ as the eluant was used to separate the desired product from starting materials and monoadduct. The resulting yellow oil (1.5 g, 43%) could not be induced to crystallize and was characterized by TLC, field desorption mass spectroscopy, NMR, and IR.

Preparation of 4'-amino-3,5-bis[N,N-bis(t-butoxycarbonylmethyl)aminomethyl]-4-methoxybenzophenone (5)

Nitrotetraester *4* (3.0 g, 3.9 mmol) was reduced in 50 ml $CH_3OH$ with 100 mg 10% Pd/C in a Parr shaker with an initial hydrogen pressure of 50 psi for 2.5 hours. TLC indicated quantitative reduction. The mixture was filtered through diatomaceous earth and evaporated to leave a yellow oil that was purified by gel permeation chromatography by the method used for compound *4*. The yellow glass thus obtained had the correct NMR, IR, and field desorption mass spectroscopic behavior.

Preparation of 3,5-bis[N,N-bis-t-butoxycarbonylmethyl)aminomethyl]-4'-{N'-[4-(4-hydroxy-3,5-diiodophenoxy)-3,5-diiodo-β-methoxycarbonylphenethyl]thioureido}-4-methoxybenzophenone (6)

Tetraester amine *5* (1.7 g, 2.3 mmol) together with triethylamine (1.28 ml, 9.2 mmol) was dissolved in 40 ml of dry tetrahydrofuran (THF), followed by the dropwise addition of thiophosgene (0.175 ml, 2.3 mmol) in 10 ml of dry THF. The reaction was allowed to proceed for 2 hours, after which the solvent was removed to yield a yellow-orange oil. The oil was dissolved in 60 ml dry N,N-dimethylformamide (DMF) and a solution of L-thyroxine methyl ester hydrochloride (1.9 g, 2.3 mmol) and triethylamine (0.32 ml, 2.3 mmol) in 20 ml DMF was added in one portion. The reaction was stirred for 1 hour under $N_2$, then poured into 350 ml $H_2O$. Extraction with ether followed by the ether layer being successively washed with three 300-ml portions of purified water, dried over $Na_2SO_4$, filtered, and evaporated gave 3.1 g of orange-white foam. This material was purified by gel permeation chromatography to give 1.3 g of the desired product as an orange-white foam. The product was further characterized by TLC, NMR, and field desorption mass spectroscopy.

Preparation and evaluation of 3,5-bis[N,N-bis(carboxymethyl)aminomethyl]-4'-{N'-[4-(4-hydroxy-3,5-diiodophenoxy)-3,5-diiodo-β-methoxycarbonylphenethyl]thioureido}-4-methoxybenzophenone (7)

Tetraester *6* (0.5 g, 0.3 mmol) was dissolved in 15 ml $CF_3CO_2H$ and stirred for 16 hours at room temperature with a drying tube attached to the reaction flask. The solvent was removed *in vacuo* to leave an orange foam. Trituration of the foam with $CH_2Cl_2$ produced a yellow-orange powder in quantitative yield. The field desorption mass spectrum shows a parent ion at m/e (mass/charge ratio) 1350 and the IR is consistent with the product.

Anal. Calc'd for

| | | | | |
|---|---|---|---|---|
| $C_{41}H_{38}I_4N_4O_{14}S$: | C, 36.5; | H, 2.8; | N, 4.1; | S, 2.4. |
| Found: | C, 36.4; | H, 2.8; | N, 3.7; | S, 2.7. |

One equivalent of the above compound and two equivalents of $EuCl_3 \cdot 6H_2O$ in pH 8.5 borate buffer were weakly fluorescent under long-wavelength UV light giving the characteristic red emission. A linear relationship between fluorescence intensity and chelate concentration was demonstrated between $10^{-5}$ and $10^{-7}$ M with the Varian SF330 (trade mark) Spectrofluorimeter and λex = 320 nm, λem = 614 nm. One equivalent of compound *7* and two equivalents of $TbCl_3 \cdot 6H_2O$ in pH 8.5 borate buffer were strongly fluorescent under long-wavelength UV light. This chelate also had a linear relationship between fluorescence intensity and chelate concentration between $10^{-5}$ M and $5 \times 10^{-8}$ M.

Cross reactivity is a reflection of how well an antibody recognizes the labeled antigen as compared with unlabeled antigen. The cross reactivity of the europium chelate of the antigen as determined by known techniques was 75% vs. radiolabeled thyroxine and thyroxine antibody.

Example 9

Complex of europium with 3,5-bis[N,N-bis(carboxymethyl)aminomethyl]-4-hydroxy-3'-[4-(4-hydroxy-3,5-diiodophenoxy)-3,5-diiodo-β-ethoxycarbonylphenethyl]benzophenone

The synthetic scheme for the preparation of the chelating agent of this example is as follows:

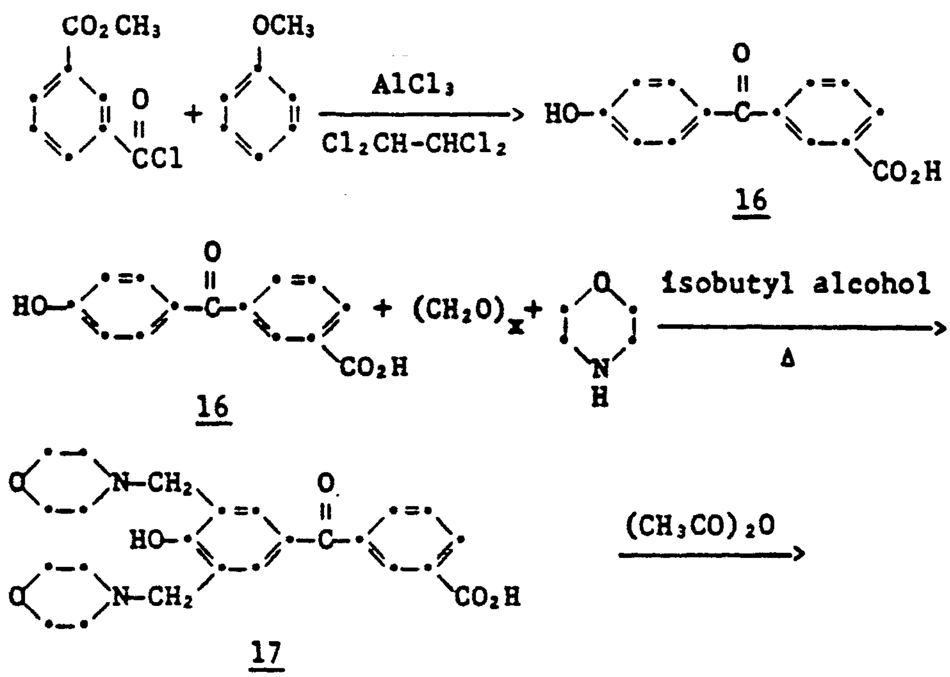

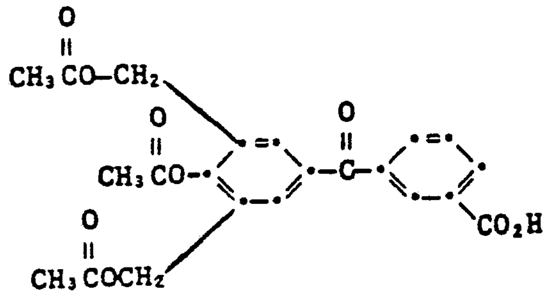

18

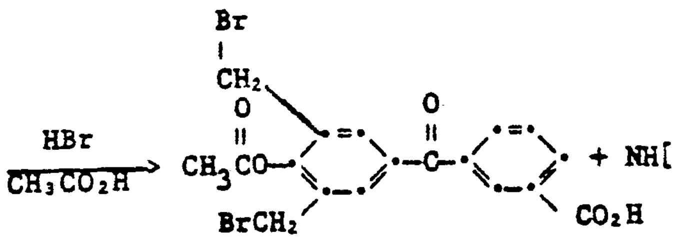


+ $NH[CH_2CO_2C(CH_3)_3]_2$ → 20

19

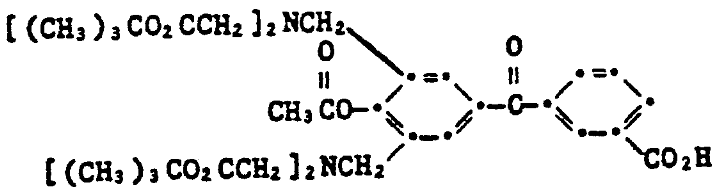

+

20

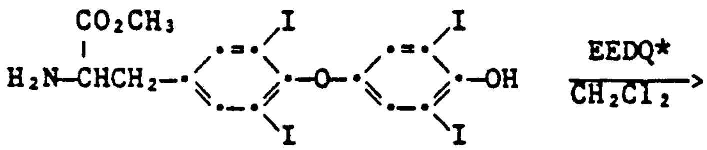


*EEDQ = 1-carbethoxy-1-ethoxy-1,2-dihydroquinoline

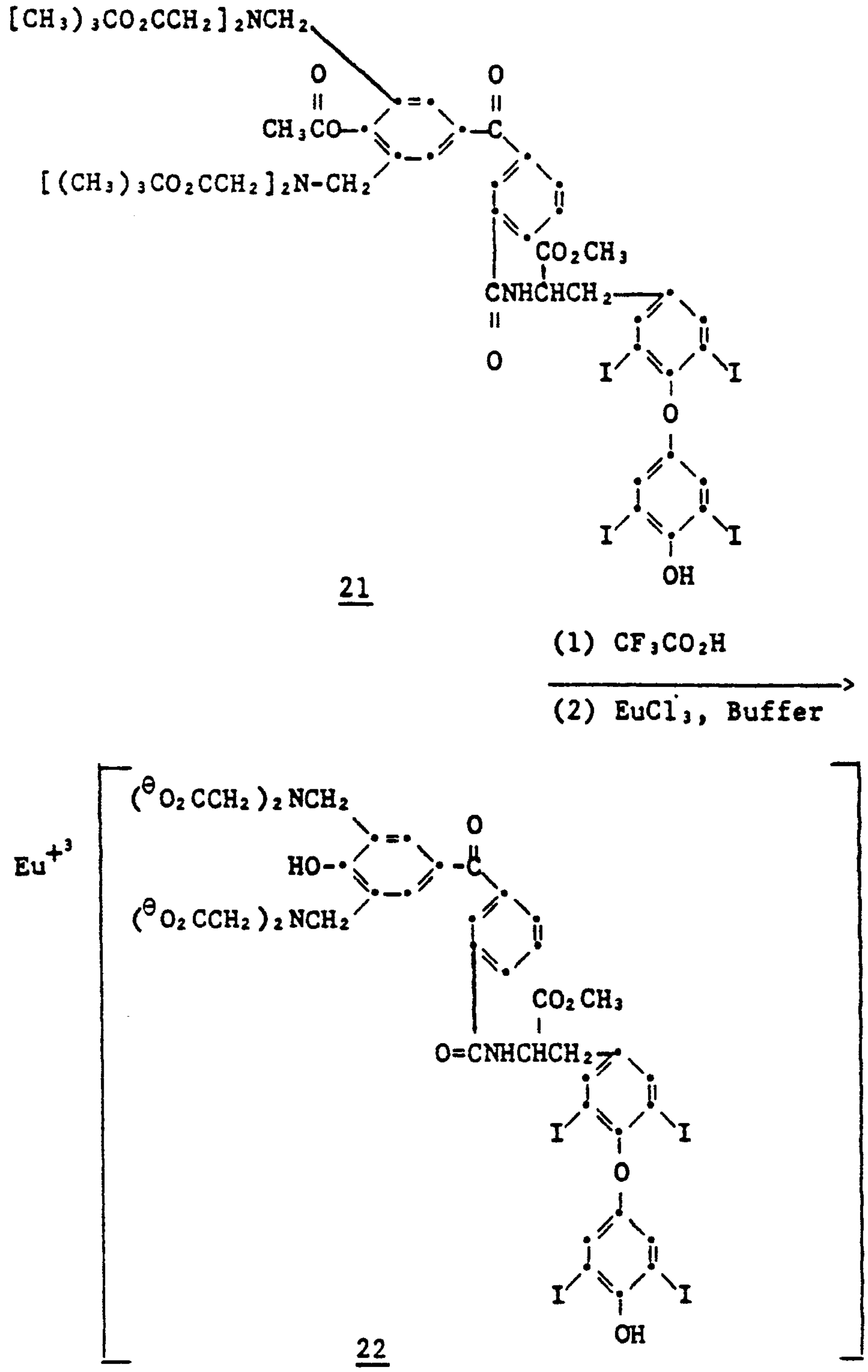


21

22

3-Carboxy-4'-hydroxybenzophenone (16)

This compound was prepared by the method described in U.S. Patent 3,531,435 (*Chem. Abstracts 74,* 4283V (1971)). A solution containing 26.3 g of anisole (0.24 mole) and 46 g of 3-carbomethoxybenzoyl chloride (0.23 mole) in 10 ml of tetrachloroethane was added dropwise over 1 hour to a stirred solution of 65 g $AlCl_3$ (0.49 mole) in 100 ml tetrachloroethane at 0°C. The mixture was allowed to come to room temperature and was stirred for 14 hours. The temperature was then raised to 75—80°C and 32 g of $AlCl_3$ (0.24 mole) was added over a 30 minute period. The temperature was maintained for 45 minutes, then the solution was poured into a mixture of ice and 64 ml of concentrated HCl. The lower phase was removed and washed with water. The tetrachloroethane was removed by steam distillation leaving a solid brown residue that was collected and recrystallized from tetrahydrofurane and toluene. Saponification of this intermediate with aqueous NaOH gave, after acidification, the desired product as a white powder (16.5 g, 29%): mp 240—241°C (lit. 240—241°C):

| | | |
|---|---|---|
| Anal. Calc'd for $C_{14}H_{10}O_4$: | C, 69.4; | H, 4.2. |
| Found: | C, 69.5; | H, 4.4. |

3'-Carboxy-3,5-bis(morpholinomethyl)-4-hydroxybenzophenone (17)

A mixture of 5.22 g (0.06 mole) of morpholine and 1.8 g (0.06 mole) of paraformaldehyde in 50 ml of isobutyl alcohol was refluxed under nitrogen until a clear solution was obtained. To this solution was added 4.8 g (0.02 mole) of 3-carboxy-4'-hydroxybenzophenone (*16*) and the refluxing was continued for 3 hours. The solution was evaporated under reduced pressure and the gummy residue was stirred several times with ether, giving 9.5 g of solid, which was not purified.

4-Acetoxy-3,5-bis(acetoxymethyl)-3'-carboxybenzophenone (18)

A mixture of 9.5 g of *17* and 75 ml of acetic anhydride was refluxed for 24 hours and the excess acetic anhydride was removed under vacuum. The residue was stirred with water and the solid was collected and dried; yield 8.5 g. Thin-layer chromatography (silica gel; 1% methanol in methylene chloride) shows about 10% of the faster moving monoacetoxymethyl derivative is present in the *18.*

4-Acetoxy-3,5-bisbromomethyl-3'-carboxybenzophenone (19)

A solution of 2 g of *18,* 20 ml of methylene chloride and 5 ml of 31% hydrobromic acid in acetic acid was stirred overnight, 3 ml of acetic anhydride was added, and the solution was evaporated to dryness. The residue was chromatographed on silica gel eluting with 1:1 $CH_3CO_2C_2H_5/CH_2Cl_2$ using the method of Still (W. C. Still, M. Kahn and A. Mitra, *J Org Chem, 43,* 2923 (1978)), giving 0.56 g of pure *19* as determined by NMR.

4-Acetoxy-3,5-bis[N,N-bis(t-butoxycarbonylmethyl)aminomethyl]-3'-carboxybenzophenone (20)

A solution of 460 mg (1.07 mmol) of *19,* 524 mg (2.14 mmol) of *tert*-butyl iminodiacetate, and 216 mg (2.14 mmol) of triethylamine in 15 ml of dry tetrahydrofuran was stirred under argon for 2 days. The reaction mixture was filtered to remove triethylamine hydrobromide. The filtrate was evaporated to dryness, giving 90 mg of *20.*

4-Acetoxy-3,5-bis[N,N-bis(t-butoxycarbonylmethyl)aminomethyl]-3'-[4-(4-hydroxy-3,5-diiodophenoxy)-3,5-diiodo-β-methoxycarbonylphenethyl]benzophenone (21)

A mixture of 900 mg of *20* and 0.265 g (1.07 mmol) of 1-carbethoxy-2-ethoxy-1,2-dihydroquinoline (EEDQ) in 20 ml of dry tetrahydrofuran was stirred for 30 minutes and 0.85 g (1.07 mmol) of the methyl ester of thyroxine was added. The mixture was stirred overnight. The reaction mixture was purified by gel permeation chromatography using tetrahydrofuran as the solvent, giving 630 mg of material which showed an NMR spectrum that was consistent with structure *21.*

3,5-bis[N,N-bis(carboxymethyl)aminomethyl]-4-hydroxy-3'-[4-(4-hydroxy-3,5-diiodophenoxy)-3,5-diiodo-β-methoxycarbonylphenethyl]benzophenone (22)

A solution of 630 mg of *21* in 5 ml of trifluoroacetic acid was stirred overnight and then diluted with water. The solid that separated was collected and dried; yield 550 mg. The field desorption mass spectrum showed *22* (m/e 1305) is present, as well as some material in which the methyl ester of the thyroxine has been hydrolyzed to the acid.

One equivalent of *22* plus two 2-equivalents of $EuCl_3 \cdot 6H_2O$ were moderately fluorescent when dissolved in pH 8.5 borate buffer and examined under long-wavelength UV light. A linear plot of fluorescence intensity vs chelate concentration was generated between $10^{-5}$ and $10^{-7}$ M for this compound. All fluorescence measurements were taken with a Varian SF330 Spectrofluorimeter.

The europium chelate of *22* had a cross reactivity of 80% vs. radiolabeled thyroxine and thyroxine antibody.

Examples 10—16

Europium and terbium complexes with the following chelating agents were prepared as in Example 9 using $EuCl_3 \cdot 6H_2O$ and $TbCl_3 \cdot 6H_2O$ in borate buffer:

| Example | Chelating Agent |
|---|---|
| 10 | 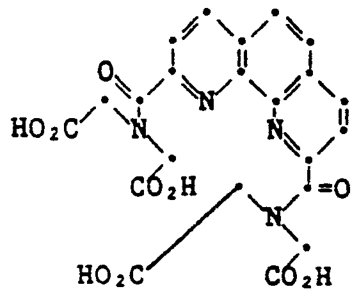 |
| 11 | 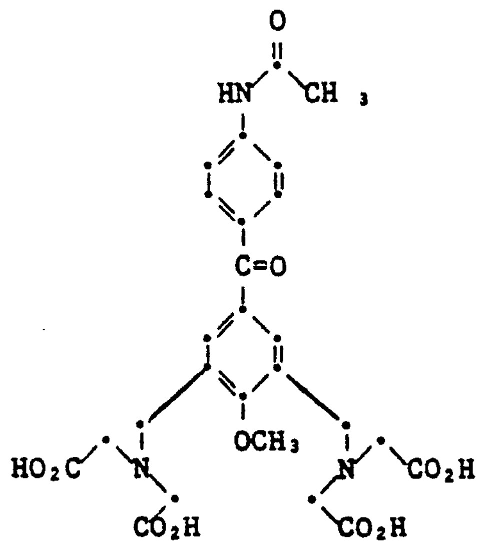 |
| 12 | 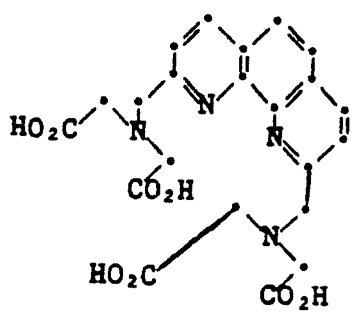 |
| 13 | 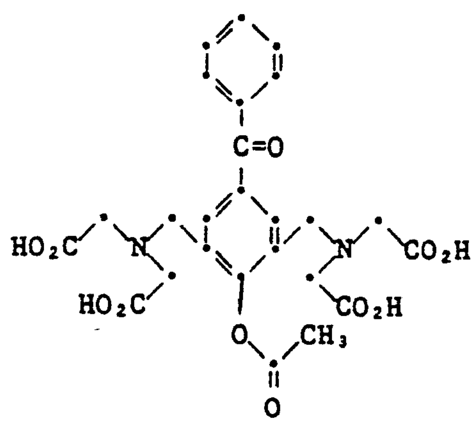 |

| Example | Chelating Agent |
|---|---|
| 14 | 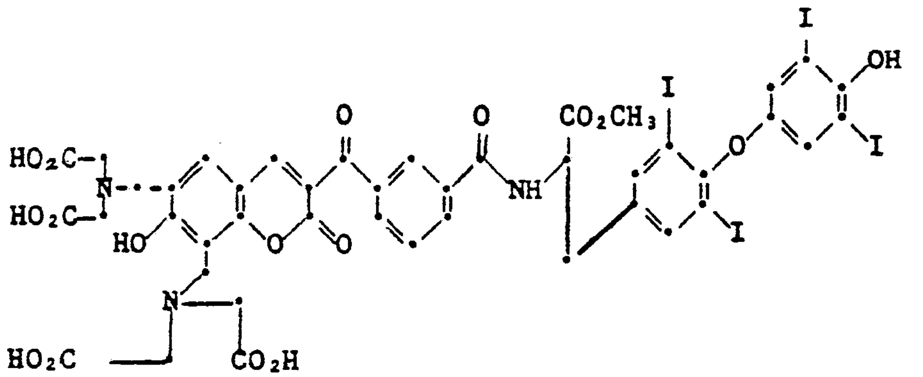 |
| 15 | 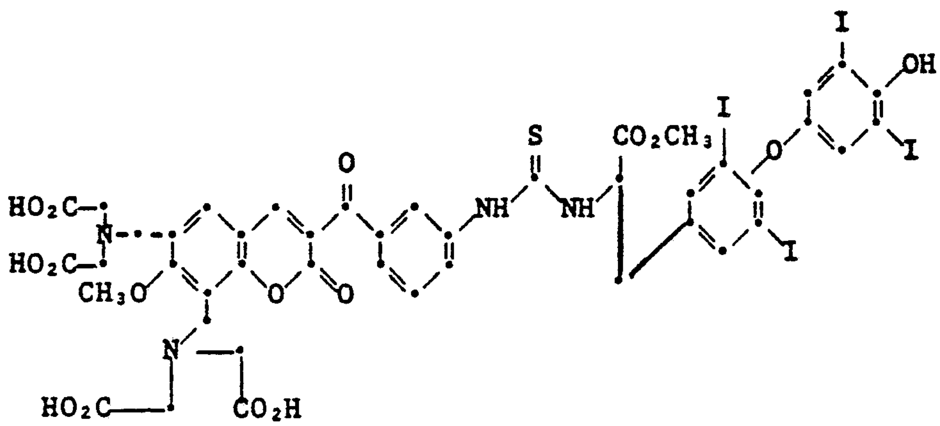 |
| 16 | 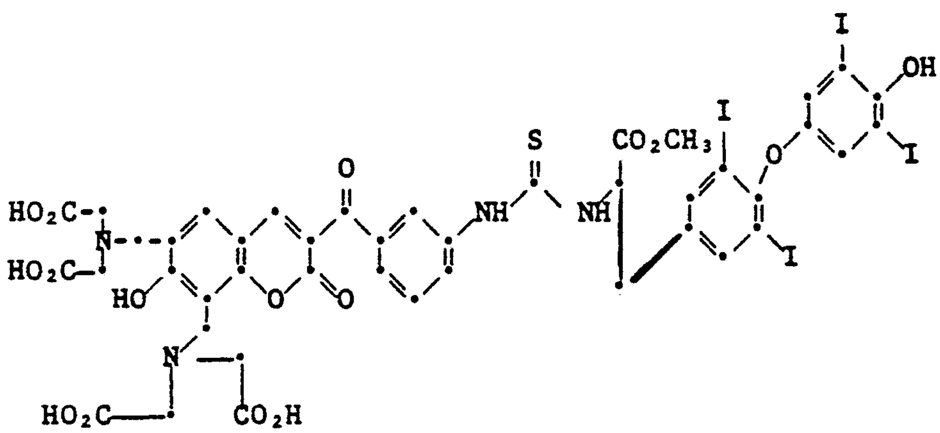 |
| Control A | 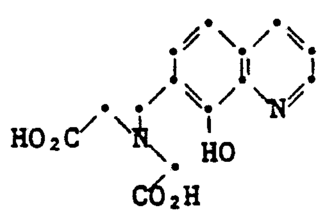 |

Control B

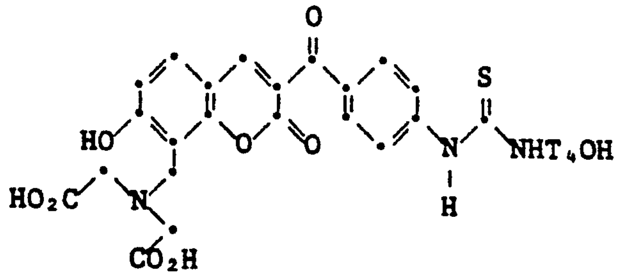

Control C

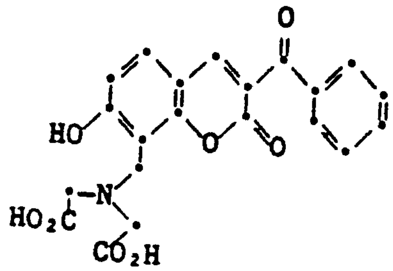

The complexes of Examples 10—16 were fluorescent and those of Controls A, B and C were not fluorescent. Complexes of Controls A, B and C were further tested in glycine acetate buffer, phosphate buffer and sodium bicarbonate buffer and were not fluorescent with either $EuCl_3 \cdot 6H_2O$ or $TbCl_3 \cdot 6H_2O$.

**Claims**

1. A fluorescent chelate comprising a lanthanide metal and a chelating agent characterised in that the chelating agent includes a moiety that is a triplet sensitizer having a triplet energy greater than that of the lanthanide metal and at least two heteroatom-containing groups and a third heteroatom-containing group or heteroatom that is in or appended to said moiety, each of said two heteroatom-containing groups appended to different carbon atoms of the triplet sensitizer moiety, the heteroatom-containing groups being located in the chelating agent such that they and said third heteroatom or heteroatom-containing group form a chelate structure with the lanthanide metal.

2. The chelate of claim 1 wherein the lanthanide metal is europium.

3. The chelate of claim 1 wherein the lanthanide metal is terbium.

4. The chelate of claim 1 wherein the mole ratio of lanthanide metal to chelating agent is from 1:1 to 2:1.

5. The chelate of any of claims 1—4 wherein the chelating agent has the structure:

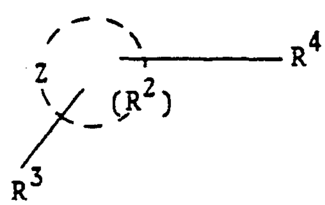

wherein:

Z together with $R^2$ represents the atoms necessary to complete a substituted or unsubstituted moiety that is a triplet sensitizer having a triplet energy greater than that of the lanthanide metal;

$R^2$ is a heteroatom or an alkylene (including alkenylene) group having at least one heteroatom therein or a heteroatom or heteroatom-containing group appended thereto; and

$R^3$ and $R^4$ are heteroatom-containing groups that are the same or different; $R^3$ and $R^4$ being in sufficient proximity to $R^2$ so that the lanthanide metal is chelatable to $R^2$, $R^3$ and $R^4$;

wherein the number of carbon and heteroatom atoms represented by $R^2$ is equal to or less than 20.

6. The chelate of claim 5 wherein the triplet sensitizer is benzophenone.

7. The chelate of any of claims 1—4 wherein the chelating agent is a phenol having iminodiacetate groups substituted in each position ortho to the phenolic hydroxy group and a carbonyl group.

8. The chelate of claim 7 wherein the substituted phenol has the structure:

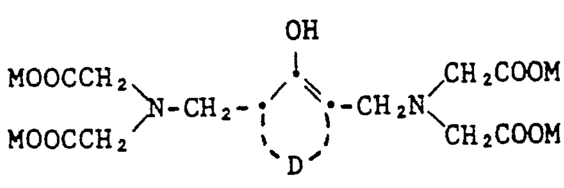

wherein:

M is hydrogen or a cation and

D represents the atoms necessary to complete a substituted or unsubstituted aromatic ring, said phenol containing a carbonyl group; or said phenol being fused at two of its available positions to another aromatic, alicyclic or heterocyclic ring, any of which may be substituted; or to a pyran ring which bears a carbonyl group to form a coumarin nucleus.

9. The chelate of claim 8 wherein the aromatic ring completed by D is phenyl substituted with a carbonyl group or a coumarin.

10. The chelate of any of claims 1—4 wherein the chelating agent is:

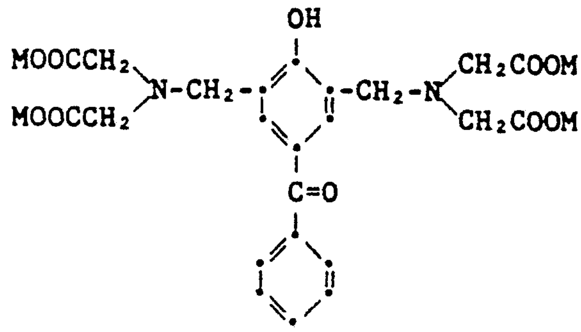

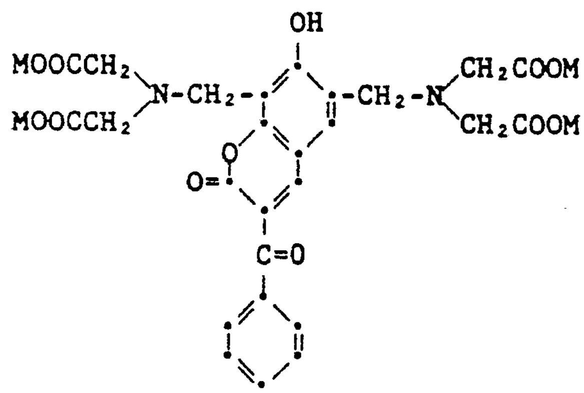

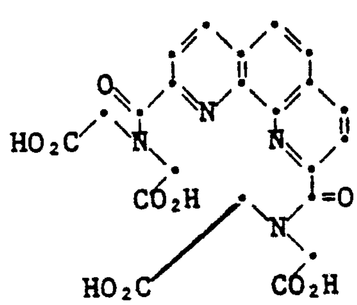

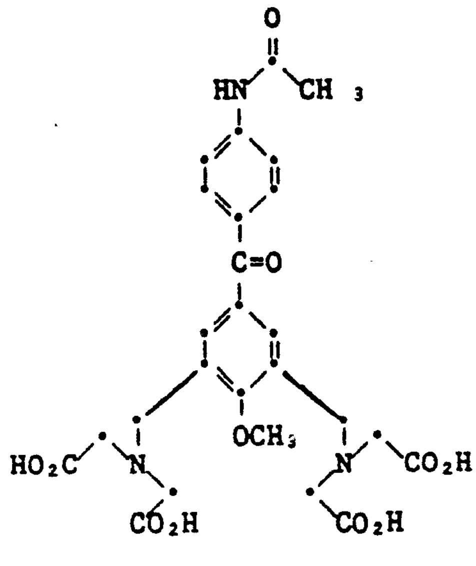

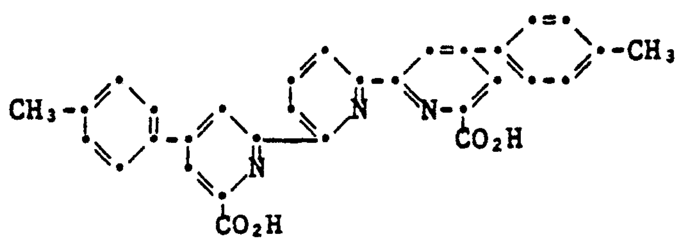

or

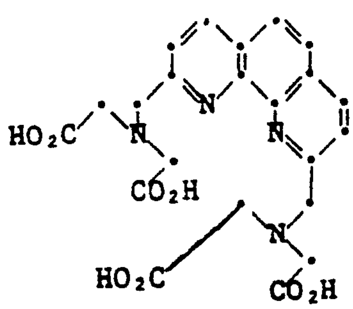

11. A fluorescently labeled specific binding reagent characterized by a physiologically active material adsorbed or bound to the fluorescent chelate of any of claims 1—10.

12. The binding reagent of claim 11 wherein the physiologically active material is an antibody, antigen, hapten, enzyme, enzyme substrate, metabolite, vitamin, hormone, hormone receptor, or lectin.

13. The binding reagent of either of claims 11 or 12 wherein the physiologically active material is bound to the fluorescent chelate through an ester, amide, sulfonamide, ether, carbonyl, nitrilo, imino, arylene, or thioarylene group.

14. The binding reagent of claim 11 having the structure:

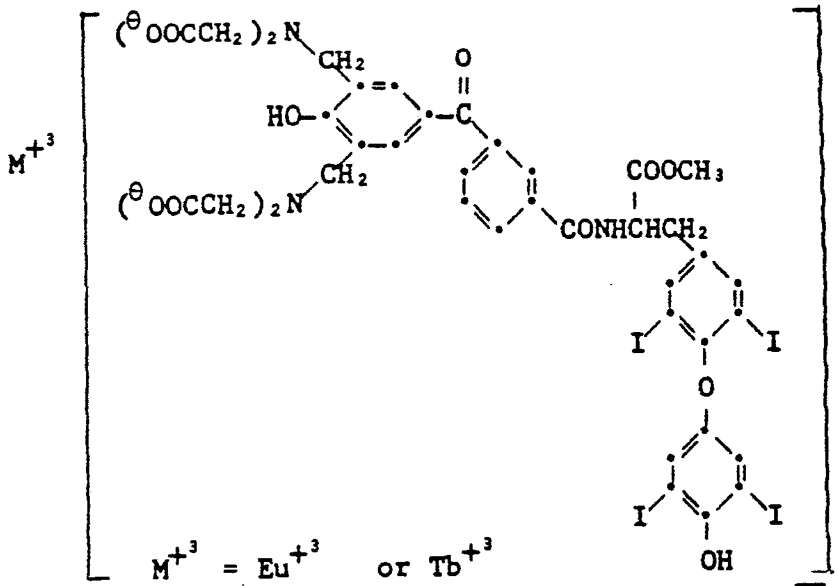

$M^{+3} = Eu^{+3}$ or $Tb^{+3}$ ,

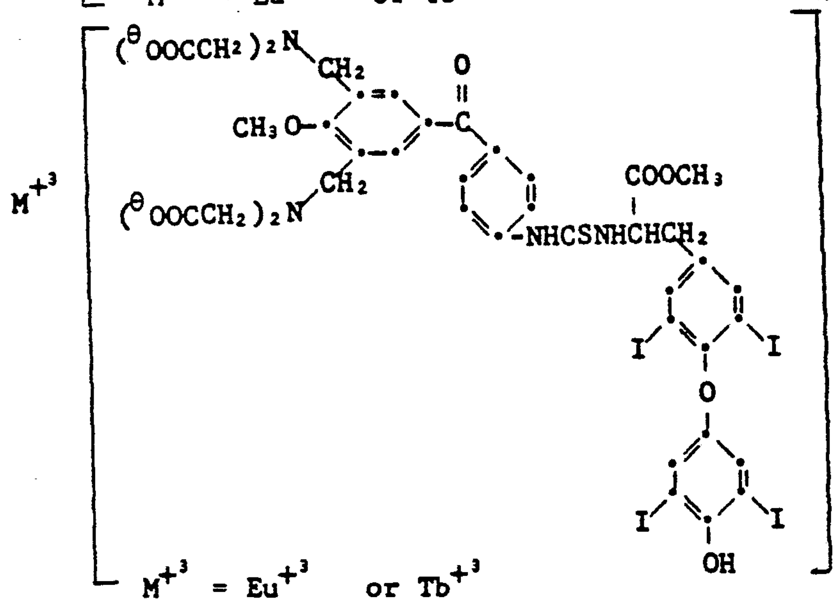

$M^{+3} = Eu^{+3}$ or $Tb^{+3}$ ,

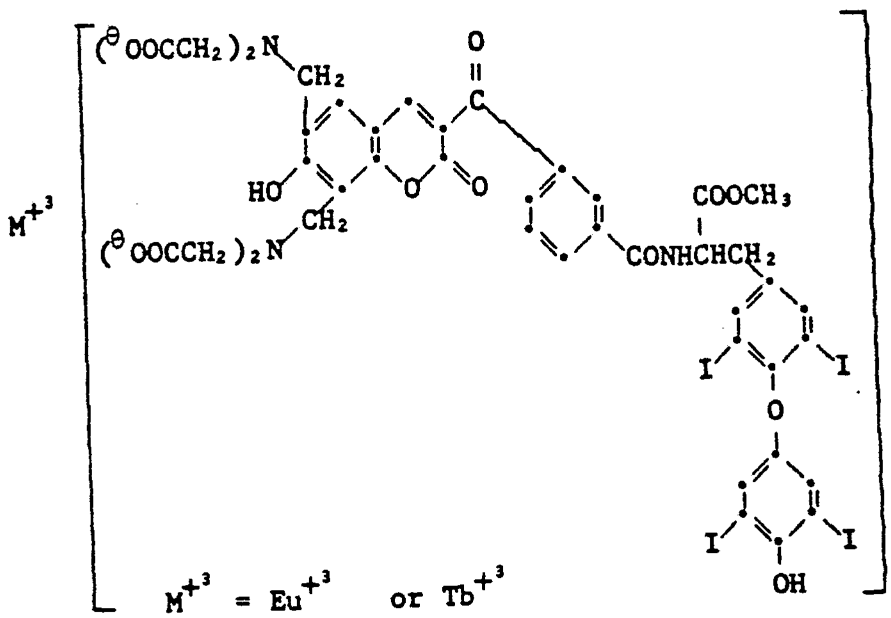

$M^{+3} = Eu^{+3}$ or $Tb^{+3}$ ,

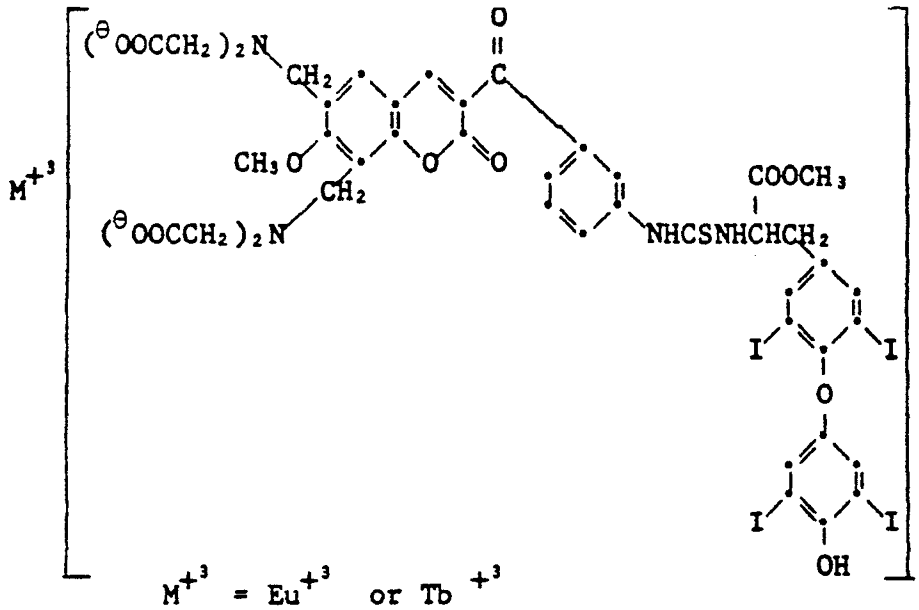

$M^{+3} = Eu^{+3}$ or $Tb^{+3}$

or

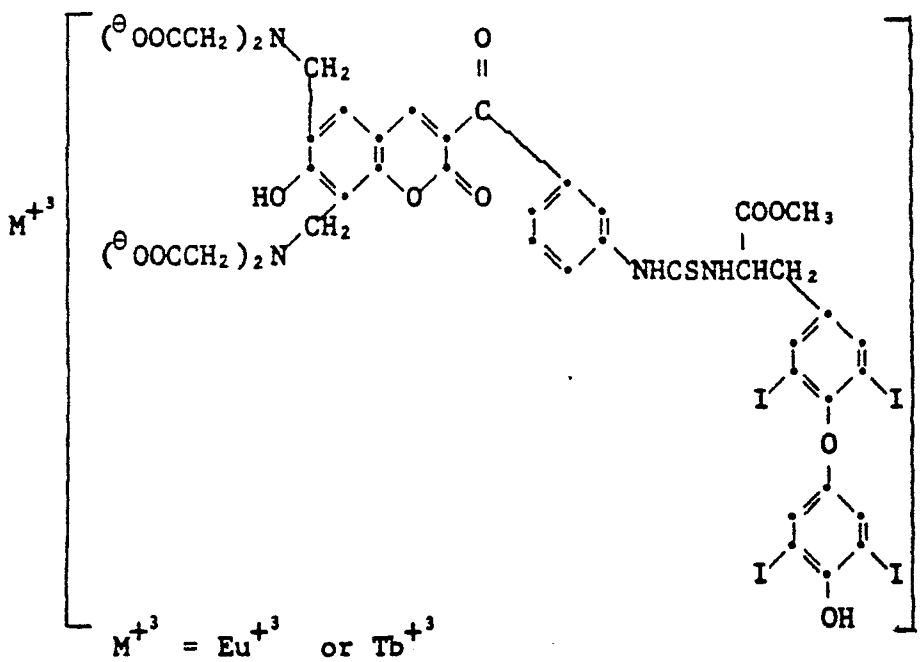

$M^{+3} = Eu^{+3}$ or $Tb^{+3}$

**Patentansprüche**

1. Fluoreszierendes Chelat aus einem Lanthanidenmetall und einem Chelatbildner, dadurch gekennzeichnet, daß der Chelatbildner einen Rest aufweist, der einen Triplet-Sensibilisator mit einer Tripletenergie darstellt, die größer ist als die des Lanthanidenmetalles, sowie mindestens zwei Heteroatom enthaltende Gruppen und eine dritte Heteroatom enthaltende Gruppe oder ein Heteroatom, das in dem Rest vorliegt oder an den Rest gebunden ist, wobei jede der zwei Heteroatom enthaltenden Gruppen an verschiedene Kohlenstoffatome des Triplet-Sensibilisatorrestes gebunden ist und wobei die Heteroatom enthaltenden Gruppen in dem Chelatbildner eine solche Position einenhmen, daß sie und das dritte Heteroatom oder Heteroatom enthaltende Gruppe eine Chelatstruktur mit dem Lanthanidenmetall bilden.

2. Chelat nach Anspruch 1, dadurch gekennzeichnet, daß das Lanthanidenmetall Europium ist.

3. Chelat nach Anspruch 1, dadurch gekennzeichnet, daß das Lanthanidenmetall Terbium ist.

4. Chelat nach Anspruch 1, dadurch gekennzeichnet, daß das Molverhältnis von Lanthanidenmetall und Chelatbildner bei 1:1 bis 2:1 liegt.

5. Chelat nach einem der Ansprüche 1 bis 4, dadurch gekennzeichnet, daß der Chelatbildner der folgenden Strukturformel entspricht:

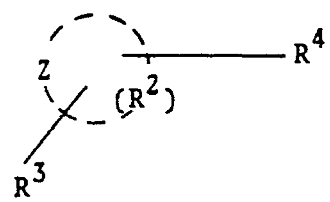

in der bedeuten:

Z gemeinsam mit $R^2$ die Atome, die zur Vervollständigung eines substituierten oder unsubstituierten Restes erforderlich sind, der einen Triplet-Sensibilisator mit einer Tripletenergie darstellt, die größer ist als die das Lanthanidenmetalles;

$R^2$ ein Heteroatom oder eine Alkylengruppe (einschließlich einer Alkenylengruppe) mit mindestens einem Heteroatom in der Gruppe oder mit einem Heteroatom oder einer Heteroatom enthaltenden Gruppe, die an die Alkylengruppe gebunden sind, und

$R^3$ und $R^4$ gleiche oder verschiedene Heteroatom enthaltende Gruppen, wobei sich $R^3$ und $R^4$ in ausreichender Näher zu $R^2$ befinden, so daß das Lanthanidenmetall durch $R^2$, $R^3$ und $R^4$ zu einem Chelat gebunden werden kann, wobei die Anzahl der Kohlenstoffatome und Heteroatome, die durch $R^2$ dargestellt werden, gleich 20 oder geringer ist.

6. Chelat nach Anspruch 5, dadurch gekennzeichnet, daß der Triplet-Sensibilisator Benzophenon ist.

7. Chelat nach einem der Ansprüche 1 bis 4, dadurch gekennzeichnet, daß der Chelatbildner ein Phenol ist, das in jeder ortho-Position durch eine Iminodiacetatgruppe und ferner durch eine Carbonylgruppe substituiert ist.

8. Chelat nach Anspruch 7, dadurch gekennzeichnet, daß das substituierte Phenol der folgenden Struktur entspricht:

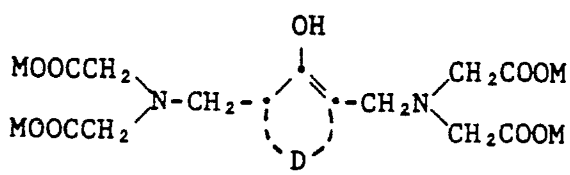

in der bedeuten:

M ein Wasserstoffatom oder ein Kation und

D die zur Vervollständigung eines substituierten oder unsubstituierten aromatischen Ringes erforderlichen Atome, wobei das Phenol eine Carbonylgruppe enthält; oder wobei das Phenol mit zwei seiner zur Verfügung stehenden Positionen an einen anderen aromatischen, alicyclischen oder heterocyclischen Ring, von denen ein jeder substituiert sein kann, oder an einen Pyranring, der unter Bildung eines Coumarinkernes eine Carbonylgruppe trägt, ankondensiert ist.

9. Chelat nach Anspruch 8, dadurch gekennzeichnet, daß der durch D vervollständigte aromatische Ring ein Phenylring, der durch eine Carbonylgruppe substituiert ist, oder ein Coumarinring ist.

10. Chelat nach einem der Ansprüche 1 bis 4, dadurch gekennzeichnet, daß der Chelatbildner besteht aus:

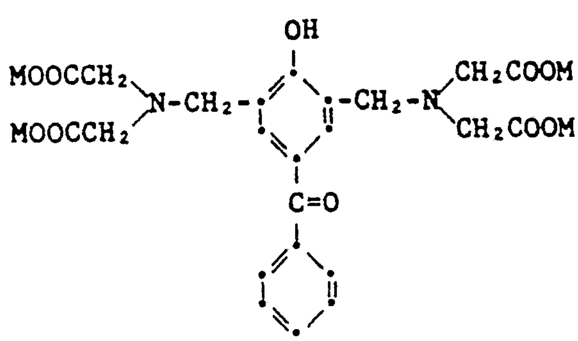

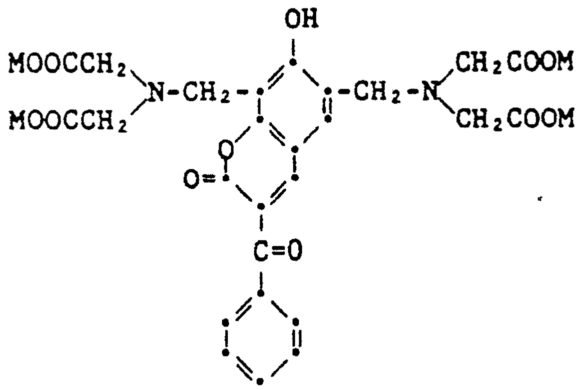

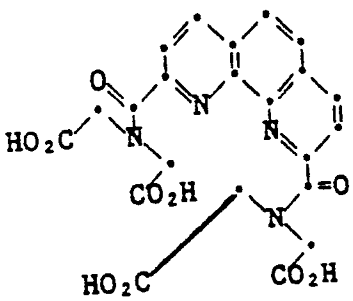

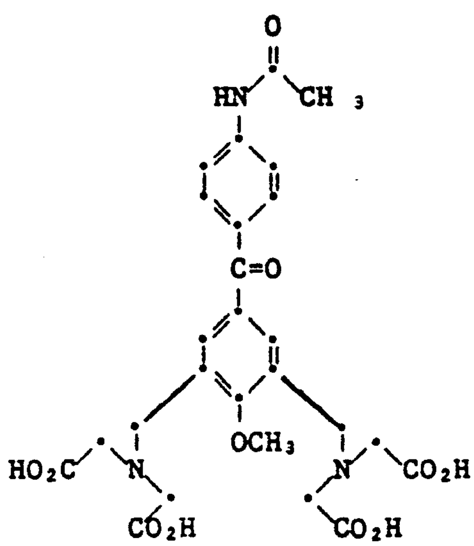

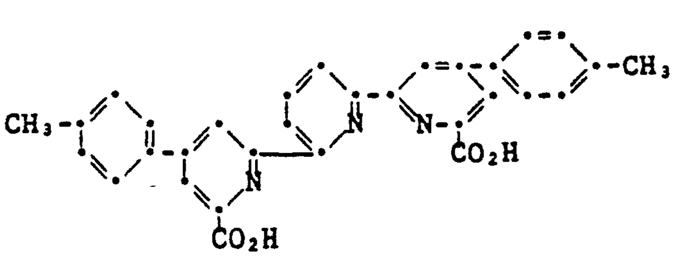

oder

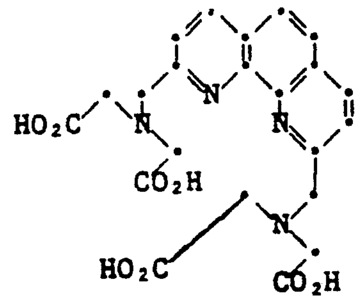

11. Fluoreszierend markiertes spezifisches Binde-Reagens, gekennzeichnet durch ein physiologisch aktives Material, das an das fluoreszierende Chelat nach einem der Ansprüche 1 bis 10 adsorbiert oder gebunden ist.

12. Binde-Reagens nach Anspruch 11, dadurch gekennzeichnet, daß das physiologisch aktive Material ein Antikörper, Antigen, Hapten, Enzym, Enzymsubstrat, Metabolit, Vitamin, Hormon, Hormonreceptor oder Lectin ist.

13. Binde-Reagens nach einem der Ansprüche 11 oder 12, dadurch gekennzeichnet, daß das physiologisch aktive Material an das fluoreszierende Chelat durch eine Ester-, Amid-, Sulfonamid-, Ether-, Carbonyl-, Nitrilo-, Imino-, Arylen- oder Thioarylengruppe gebunden ist.

14. Binde-Reagens nach Anspruch 11, gekennzeichnet durch die folgende Struktur:

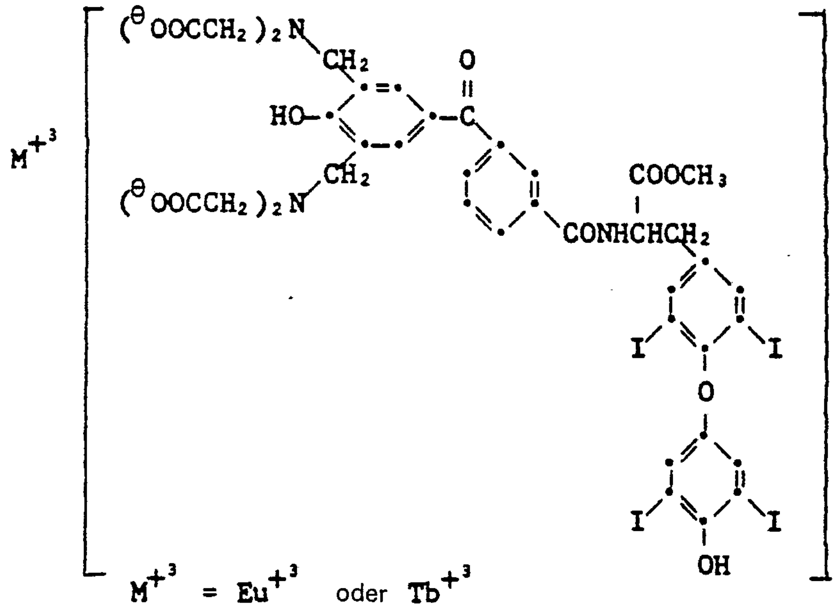

$M^{+3}$ = $Eu^{+3}$ oder $Tb^{+3}$ ,

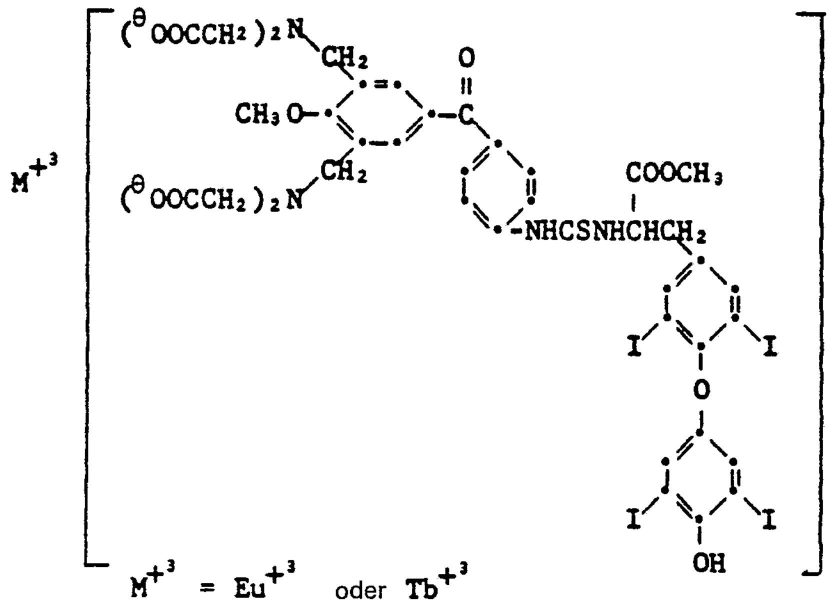

$M^{+3}$ = $Eu^{+3}$ oder $Tb^{+3}$ ,

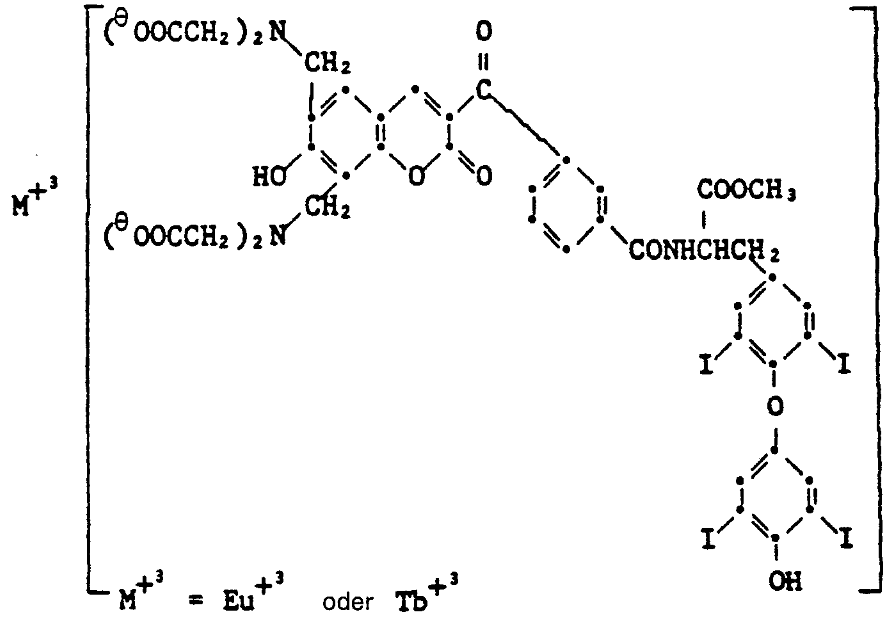

$M^{+3}$ = $Eu^{+3}$ oder $Tb^{+3}$ ,

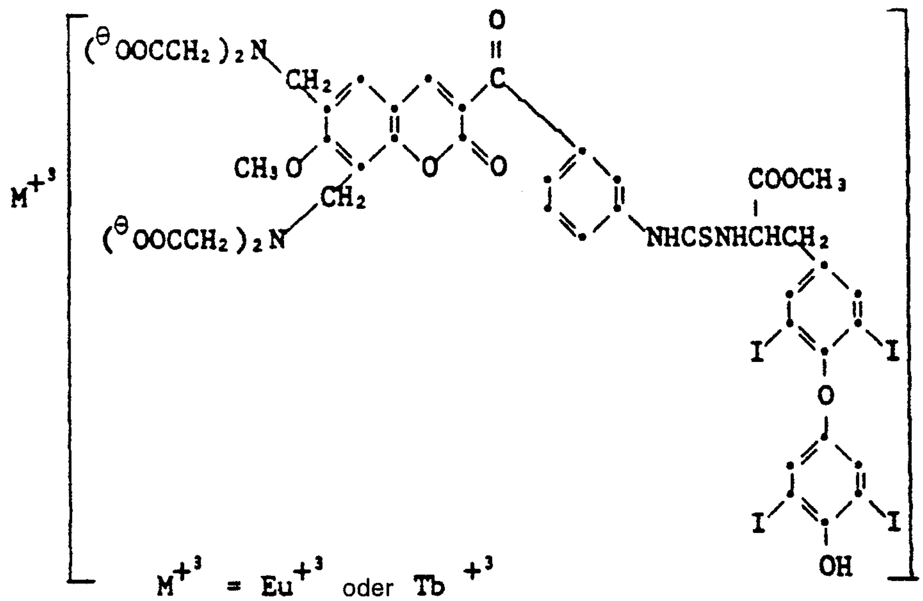

$M^{+3}$ = $Eu^{+3}$ oder $Tb^{+3}$ oder

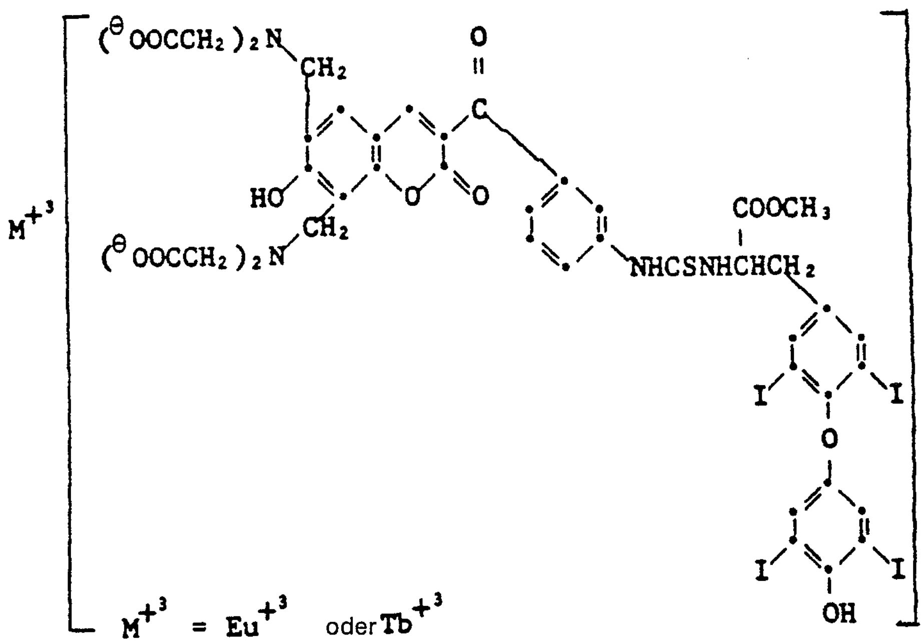

$M^{+3}$ = $Eu^{+3}$ oder $Tb^{+3}$

**Revendications**

1. Chélate fluorescent composé d'un métal de la classe des lanthanides et d'un agent chélatent, caractérisé en ce que l'agent chélatant comprend un motif qui est un sensibilisateur à l'état triplet ayant une énergie de triplet supérieure à celle du lanthanide, au moins deux groupes contenant un hétéroatome, et un troisième groupe étant ou contenant un hétéroatome, ce troisième groupe faisant partie ou étant lié au dit motif, chacun des deux groupes contenant un hétéroatome étant lié à différents atomes de carbone du motif sensibilisateur à l'état triplet, ces groupes contenant un hétéroatome étant situés dans l'agent chélatant de telle sorte qu'ils forment, ainsi que le troisième hétéroatome ou groupe contenant un hétéroatome, une structure de chélate avec le lanthanide.

2. Chélate de la revendication 1, dans lequel le lanthanide est l'europium.

3. Chélate de la revendication 1, dans lequel le lanthanide est le terbium.

4. Chélate de la revendication 1, dans lequel le rapport molaire du lanthanide à l'agent chélatant est compris entre 1:1 et 2:1.

5. Chélate de l'une quelconque des revendications 1 à 4, dans lequel l'agent chélatant a la structure:

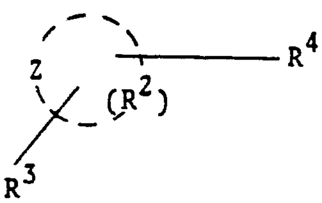

dans laquelle

Z avec $R^2$ représente les atomes nécessaires pour compléter un motif, substitué ou non, qui est un sensibilisateur à l'état triplet ayant une énergie de triplet supérieure à celle du lanthanide;

$R^2$ est un hétéroatome ou un groupe alkylène (y compris alkénylène) comprenant au moins un hétéroatome, ou auquel est lié un hétéroatome ou un groupe comprenant un hétéroatome;

$R^3$ et $R^4$ sont des groupes contenant des hétéroatomes, identiques ou différents; $R^3$ et $R^4$ étant suffisamment proches de $R^2$ pour que le lanthanide soit chélatable par $R^2$, $R^3$ et $R^4$;
le nombre d'atomes de carbone et d'hétéroatomes représentés par $R^2$ étant égal ou inférieur à 20.

6. Chélate de la revendication 5, dans lequel le sensibilisateur à l'état triplet est la benzophénone.

7. Chélate d'une quelconque des revendications 1 à 4, dans lequel l'agent chélatant est un phénol comprenant, substitués sur chacune des positions ortho du groupe hydroxy phénolique, des groupes iminodiacétate, et un groupe carbonyle.

8. Chélate de la revendication 7, dans lequel le phénol substitué à la formule:

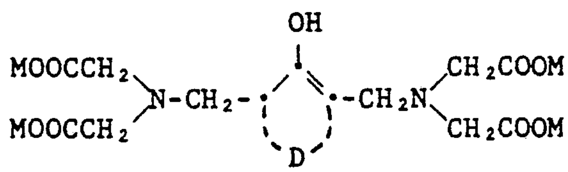

dans laquelle:

M est un atome d'hydrogéne ou un cation;

D représente les atomes nécessaires pour compléter un noyau aromatique substitué ou non, contenant un groupe carbonyle; ou le dit phénol étant condensé sur deux de ses positions disponibles avec un autre noyau aromatique, alicyclique ou hétérocyclique, chacun d'eux pouvant être substitué; ou avec un noyau pyranne portant un groupe carbonyle pour former un noyau coumarine.

9. Chélate de la revendication 8, dans lequel le noyau aromatique complété par D est un groupe phényle substitué avec un groupe carbonyle ou une coumarine.

10. Chélate de l'une quelconque des revendications 1 à 4, dans lequel l'agent chélatant est:

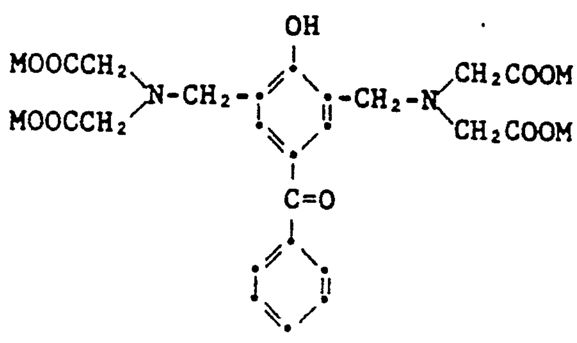

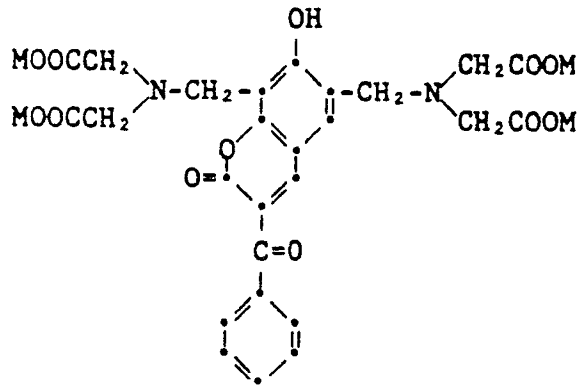

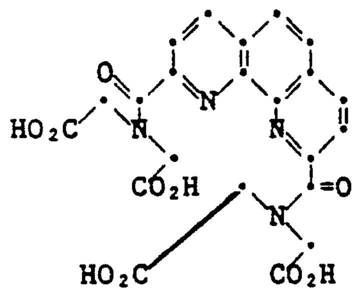

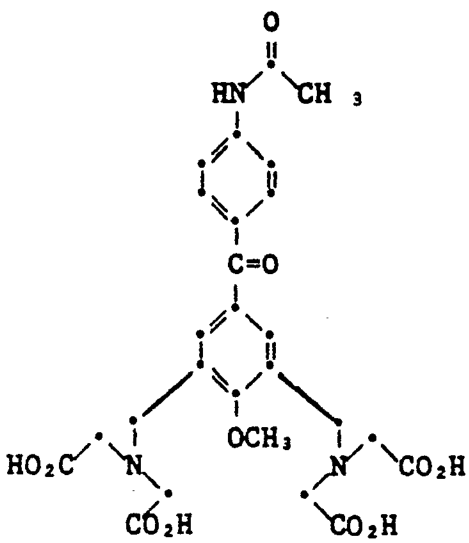

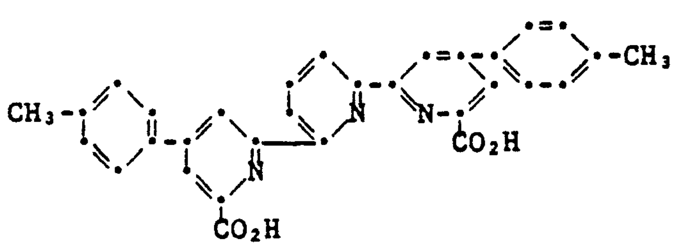

ou

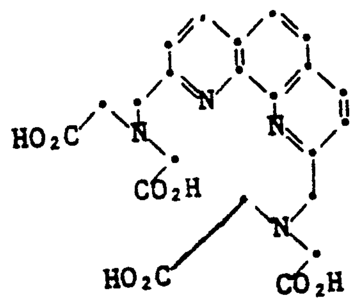

11. Réactif liant spécifique marqué par fluorescence, caractérisé en ce qu'il est constitué d'une substance physiologiquement active adsorbée ou liée sur le chélate fluorescent de l'une quelconque des revendications 1 à 10.

12. Réactif liant de la revendication 11, dans lequel la substance physiologiquement active est un anticorps, un antigène, un haptène, une enzyme, un substrat pour une enzyme, un métabolite, une vitamine, une hormone, un récepteur d'hormone, ou une lectine.

13. Réactif liant de l'une ou l'autre des revendications 11 ou 12, dans lequel la substance physiologiquement active est liée au chélate fluorescent par l'intermédiaire d'un groupe ester, amide, sulfonamide, éther, carbonyle, nitrilo, imino, arylène, ou thioarylène.

14. Réactif liant de la revendication 11, ayant la structure:

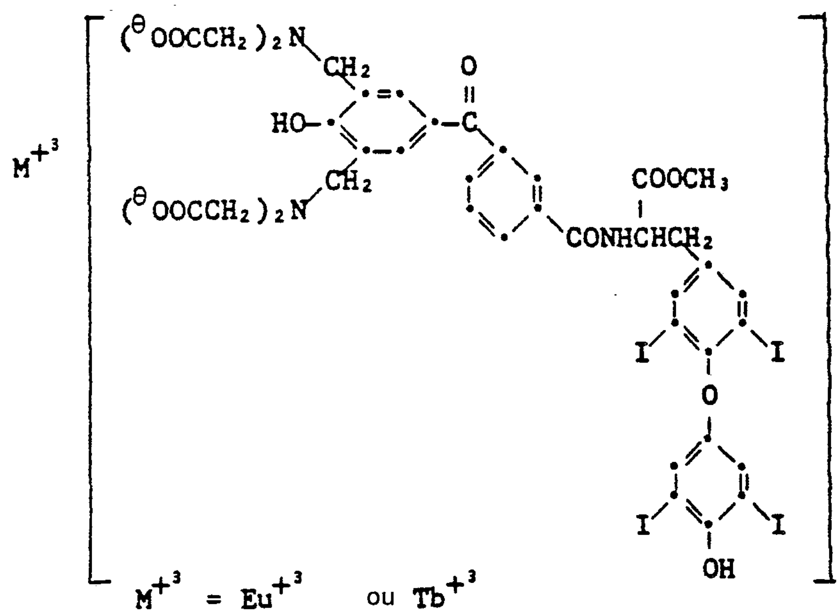

$M^{+3}$ = $Eu^{+3}$ ou $Tb^{+3}$ ,

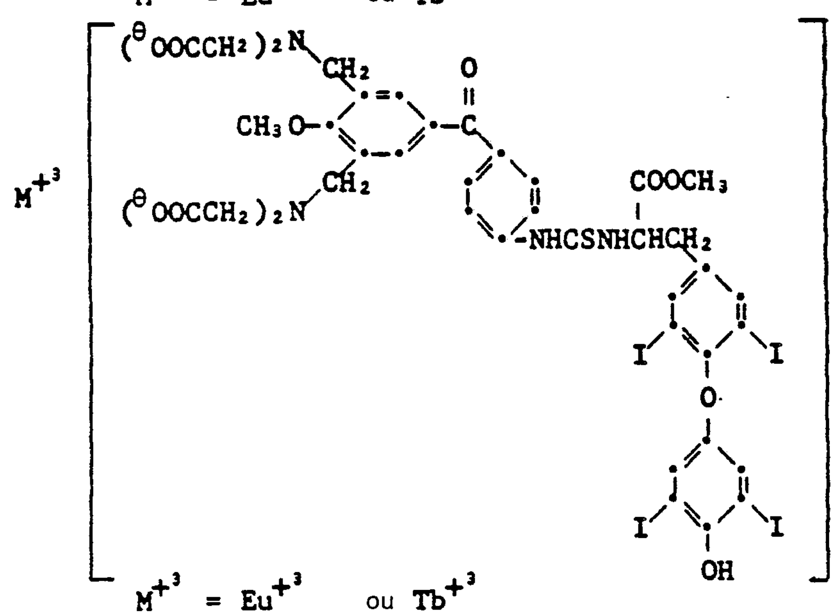

$M^{+3}$ = $Eu^{+3}$ ou $Tb^{+3}$ ,

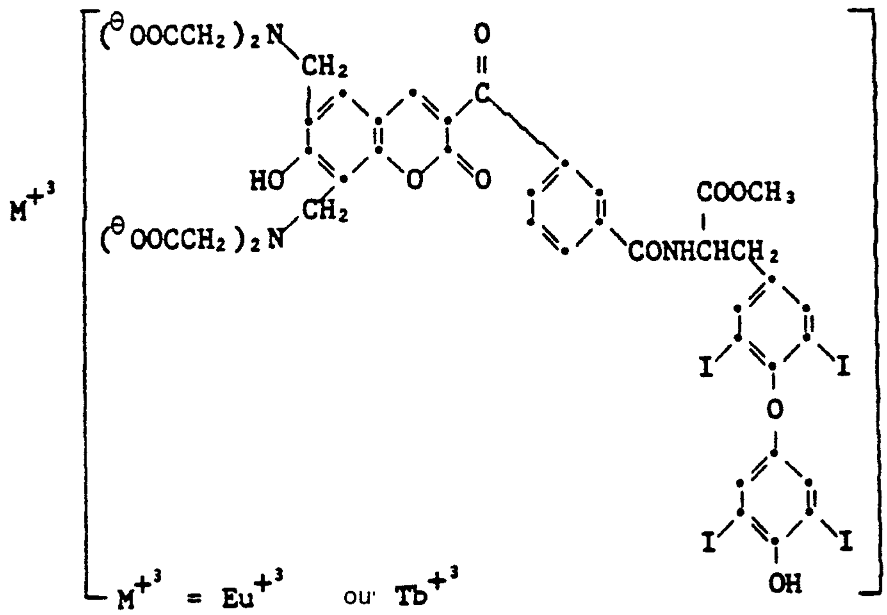

$M^{+3} = Eu^{+3}$ ou $Tb^{+3}$ ,

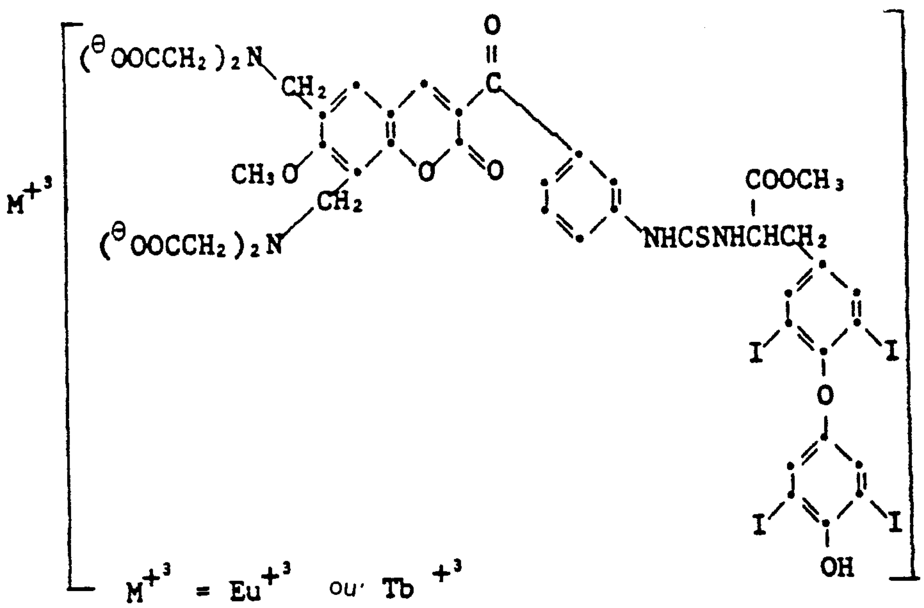

$M^{+3} = Eu^{+3}$ ou $Tb^{+3}$

ou

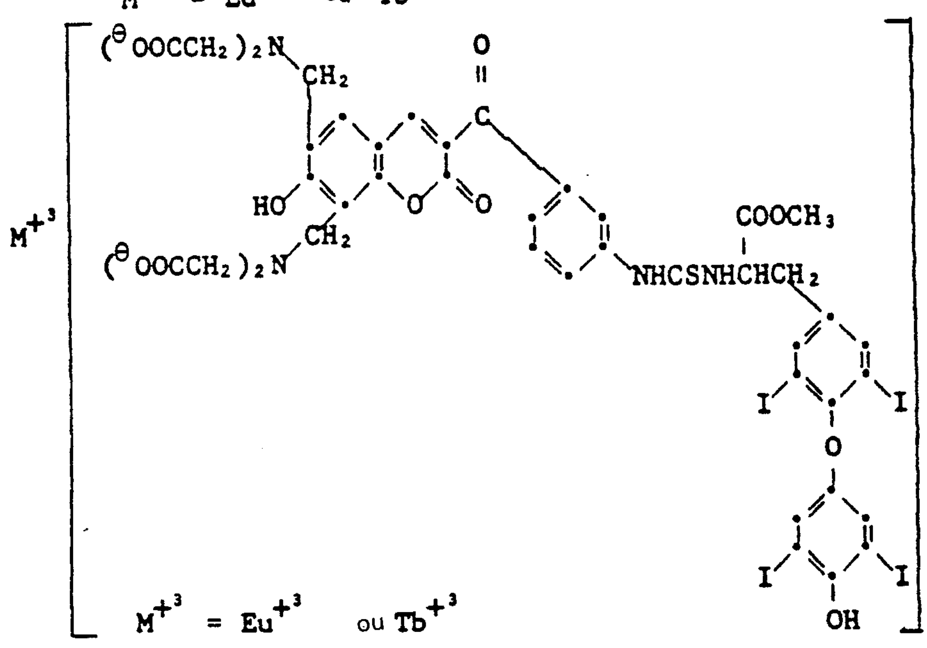

$M^{+3} = Eu^{+3}$ ou $Tb^{+3}$